# EUROPEAN PATENT SPECIFICATION

(11) **EP 4 559 914 B1**
(45) Date of publication and mention of the grant of the patent: **18.03.2026**
(21) Application number: 23842840.3
(22) Date of filing: 06.07.2023
(51) Int. Cl.: C07D 471/04, G09F 9/30, H10K 50/10, H10K 50/16, H10K 59/00, C09K 11/06, H10K 85/60

(54) **COMPOUND, LIGHT-EMITTING ELEMENT, DISPLAY DEVICE, AND LIGHTING DEVICE**
VERBINDUNG, LICHTEMITTIERENDES ELEMENT, ANZEIGEVORRICHTUNG UND BELEUCHTUNGSVORRICHTUNG
COMPOSÉ, ÉLÉMENT ÉLECTROLUMINESCENT, DISPOSITIF D'AFFICHAGE ET DISPOSITIF D'ÉCLAIRAGE

(30) Priority: 20.07.2022 JP 2022115279
(43) Date of publication of application: 28.05.2025
(73) Proprietor: Toray Industries, Inc., Tokyo 103-8666 (JP)
(72) Inventor: KOTANI Ryota, Otsu-shi, Shiga 520-8558 (JP); TOKUDA Takashi, Otsu-shi, Shiga 520-8558 (JP); NAGAO Kazumasa, Otsu-shi, Shiga 520-8558 (JP)
(74) Representative: Hoefer & Partner Patentanwälte mbB
(86) International application number: PCT/JP2023/025152
(87) International publication number: WO 2024/018915

(56) References cited:
- WO-A1-2009/054253
- WO-A1-2009/151039
- WO-A1-2021/152940
- CN-A- 112 500 330
- CN-A- 112 500 330
- KR-A- 20180 059 286
- KR-A- 20190 053 562

## Description

### TECHNICAL FIELD

The present invention relates to a compound having a specific structure, and a light-emitting element, a display device, and an illumination device using the compound.

### BACKGROUND ART

In recent years, organic EL elements (organic electroluminescent elements), which are light-emitting elements, have been steadily put into practical use, such as being employed in displays of televisions and smartphones. However, existing organic EL elements still have many technical problems. In particular, to attain both obtaining highly efficient light emission and prolonging the life of an organic EL element is a great problem.

As a compound that solves such problems, a bipyridine derivative having a bipyridine skeleton and substituted with a specific aryl group and a specific heteroaryl group (see, for example, Patent Documents 1 to 3), a bipyridine derivative substituted with an aryl group having a fluoranthene skeleton (see Patent Document 4), a bipyridine derivative having a phenanthroline skeleton (see, for example, Patent Document 5 or Patent Document 6), and the like have been developed.

### PRIOR ART DOCUMENTS

### PATENT DOCUMENTS

Patent Document 1: CN 112724073 A
Patent Document 2: CN 112500330 A
Patent Document 3: WO 2013/141097 A
Patent Document 4: WO 2015/182547 A
Patent Document 5: KR 102052332 B Patent Document 6: KR 2018 0059286 A

### SUMMARY OF THE INVENTION

### PROBLEMS TO BE SOLVED BY THE INVENTION

According to Patent Documents 1 to 5, it is possible to increase luminous efficiency by a bipyridine derivative in which a fluoranthene skeleton, a specific aryl group, a specific arylene group, or a specific heteroaryl group is linked, and obtain an organic EL element that can be driven at a low voltage and is superior in durability. However, in recent years, luminous efficiency and durability required for organic EL elements have been increasingly increased, and a technique for achieving both higher luminous efficiency and durable life has been required.

In view of the problems of the prior art, an object of the present invention is to provide a light-emitting element superior in luminous efficiency and durable life.

### SOLUTIONS TO THE PROBLEMS

In order to solve the above-described problems, the present invention provides a compound, light-emitting element, display device and illumination device as defined in the claims.

### EFFECTS OF THE INVENTION

According to the present invention, a light-emitting element superior in luminous efficiency and durable life can be provided.

### EMBODIMENTS OF THE INVENTION

Hereinafter, preferred embodiments of a compound, a light-emitting element, a display device, and an illumination device according to the present invention will be described in detail. However, the present invention is not limited to the following embodiments, and various modifications can be made according to the purpose and the use.

### (Compound Represented by General Formula (1))

The compound according to an embodiment of the present invention is a compound represented by general formula (1).

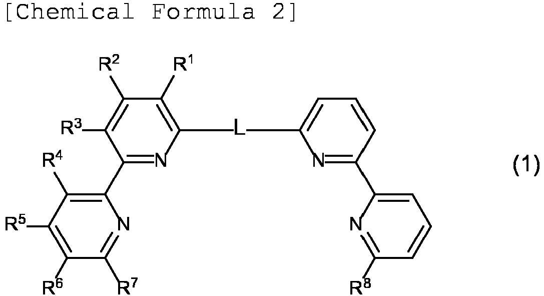

In the general formula (1), R¹, R², and R⁵ to R⁸ are each independently selected from the group consisting of a hydrogen atom, a substituted or unsubstituted alkyl group, a substituted or unsubstituted alkoxy group, a substituted or unsubstituted aryl group, and a substituted or unsubstituted heteroaryl group; R³ and R⁴ are each carbon atom having a substituent group; R³ and R⁴ may bond to each other; and L is an unsubstituted arylene group having 6 to 20 carbon atoms.

Here, the problems to be solved by the present invention will be described in detail.

As conventional compounds containing a nitrogen-containing aromatic heterocyclic ring and a polycyclic aromatic hydrocarbon, for example, Patent Documents 1 to 5 disclose compounds V, W, X, Y, and Z represented by the following formulas.

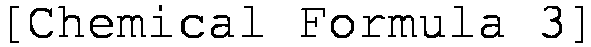

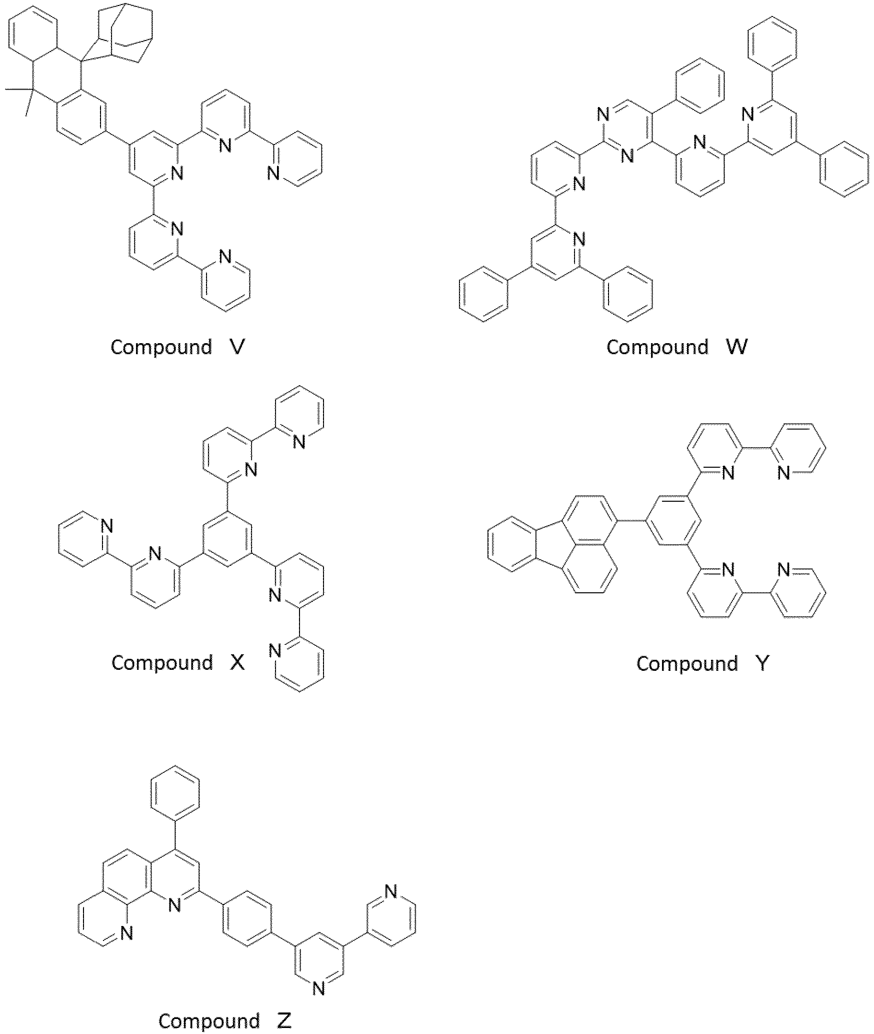

However, even in elements in which these compounds are used for an electron injection layer, an electron transporting layer, or a charge generation layer as a material for an organic EL element, sufficient performance has not yet been obtained for characteristics required in recent years. Therefore, a compound capable of further improving performance in terms of luminous efficiency and durable life is required.

For example, a heteroaryl derivative in which five consecutive pyridines and pyrimidines are linked, such as the compounds V and W, or a compound having three bipyridyl groups, such as compound X, has a problem that since a hydrogen bonding network between molecules is excessively strong, crystallinity increases, leading to deterioration of handleability and film quality stability.

A compound having a bipyridyl group and a fluoranthyl group, such as compound Y, has excessively high crystallinity due to the high flatness derived from a fluoranthene skeleton, and thus has low film quality stability, high driving voltage, and problems in luminous efficiency and durable life.

In a 3,3'-bipyridyl derivative such as compound Z, since the positions of the nitrogen atoms of pyridines are not the 2-, 2'-positions, there is a problem that the coordination property of bipyridine to a metal atom lowers and the driving voltage increases.

In the study of improvement thereof, the present inventors have focused on the effect of bipyridyl groups and their linking groups. The bipyridyl group is a substituent having a large electron transporting property and high coordination property to a metal atom. In the compound represented by the general formula (1), due to the fact that L is an unsubstituted arylene group having 6 to 20 carbon atoms, groups on both sides sandwiching L are easily conjugated. Therefore, the charge transporting property as a compound is further increased. In addition, since the number of carbon atoms of L is not too large, excessive increase in crystallinity is suppressed, and good film quality stability is provided. Therefore, the driving voltage in the case where the compound represented by the general formula (1) is used for a light-emitting element can be reduced, and the luminous efficiency and the durable life can be improved.

In addition, since a bipyridyl group has high coordination property to a metal atom, when the compound represented by the general formula (1) is used for a metal-doped layer, which is a layer doped with metal in a light-emitting element, a stable layer can be formed. In particular, when the compound represented by the general formula (1) is used for an electron transporting layer, an electron injection layer, or a charge generation layer, these layers are more stable and exhibit superior performance.

In the following, the compound represented by the general formula (1) will be described in detail.

In the general formula (1), R¹, R², and R⁵ to R⁸ are each independently selected from the group consisting of a hydrogen atom, a substituted or unsubstituted alkyl group, a substituted or unsubstituted alkoxy group, a substituted or unsubstituted aryl group, and a substituted or unsubstituted heteroaryl group.

The hydrogen atom may be a deuterium atom. The same applies to hydrogen atoms contained in other groups.

The alkyl group denotes a saturated aliphatic hydrocarbon group, such as a methyl group, an ethyl group, a n-propyl group, an isopropyl group, a n-butyl group, a sec-butyl group, or a tert-butyl group, and the alkyl group may or may not have a substituent. The number of carbon atoms of the alkyl group is not particularly limited, and is preferably in the range of 1 or more and 20 or less, and more preferably 1 or more and 8 or less from the viewpoints of easy availability and cost. The term "the number of carbon atoms" as used herein includes the number of carbon atoms contained in a substituent bonded to the alkyl group, and the same applies to other substituents whose number of carbon atoms is specified.

The alkoxy group denotes an alkyl group which is bonded to oxygen, such as a methoxy group, an ethoxy group, an n-propoxy group, an isopropoxy group, an n-butoxy group, a sec-butoxy group, or a tert-butoxy group, and the alkoxy group may or may not have a substituent. The number of carbon atoms in the alkoxy group is not particularly limited, and is usually in the range of 1 or more and 20 or less, and more preferably 1 or more and 8 or less from the viewpoints of easy availability and cost.

The aryl group denotes an aromatic hydrocarbon group, such as a phenyl group, a biphenyl group, a terphenyl group, a naphthyl group, a fluorenyl group, a benzofluorenyl group, a dibenzofluorenyl group, a phenanthryl group, an anthracenyl group, a benzophenanthryl group, a benzoanthracenyl group, a chrysenyl group, a pyrenyl group, a fluoranthenyl group, a triphenylenyl group, a benzofluoranthenyl group, a dibenzoanthracenyl group, a perylenyl group, or a helicenyl group. This may or may not have a substituent. Among them, a phenyl group and a biphenyl group are preferable.

The heteroaryl group denotes a cyclic aromatic group having one or a plurality of atoms other than carbon in the ring, such as a pyridyl group, a furanyl group, a thiophenyl group, a quinolinyl group, an isoquinolinyl group, a pyrazinyl group, a pyrimidyl group, a pyridazinyl group, a triazinyl group, a naphthyridinyl group, a cinnolinyl group, a phthalazinyl group, a quinoxalinyl group, a quinazolinyl group, a benzofuranyl group, a benzothiophenyl group, an indolyl group, a dibenzofuranyl group, a dibenzothiophenyl group, a carbazolyl group, a benzocarbazolyl group, a carbolinyl group, an indolocarbazolyl group, a benzofurocarbazolyl group, a benzothienocarbazolyl group, a dihydroindenocarbazolyl group, a benzoquinolinyl group, an acridinyl group, a dibenzoacridinyl group, a benzoimidazolyl group, an imidazopyridyl group, a benzoxazolyl group, a benzothiazolyl group, or a phenanthrolinyl group. The term "naphthyridinyl group" refers to any one of a 1,5-naphthyridinyl group, a 1,6-naphthyridinyl group, a 1,7-naphthyridinyl group, a 1,8-naphthyridinyl group, a 2,6-naphthyridinyl group, and a 2,7-naphthyridinyl group. The heteroaryl group may or may not have a substituent. The number of ring-forming atoms is not particularly limited, and is preferably in the range of 3 or more and 40 or less, and more preferably 3 or more and 30 or less.

From the viewpoint of easy availability of the compound, R¹, R², R⁵, and R⁶ are each preferably selected from the group consisting of a hydrogen atom, a methyl group, and a phenyl group. In addition, from the viewpoint of enhancing the film quality stability and further improving the durable life of the light-emitting element, R¹, R², R⁵, and R⁶ are each more preferably a hydrogen atom.

R⁷ is preferably a substituted or unsubstituted aryl group or a substituted or unsubstituted heteroaryl group. In this case, the highly reactive 2-position in the bipyridine skeleton is substituted, so that the stability of the compound is improved. R⁷ is more preferably a phenyl group, a tolyl group, a tert-butylphenyl group, a cyanophenyl group, a fluorophenyl group, or a pyridyl group. In this case, due to the efficient metal orientation property of the substituent, a more stable layer can be formed when the compound represented by the general formula (1) is used for the metal-doped layer. Therefore, the driving voltage can be further reduced, and the durable life can be improved. From the viewpoint of further enhancing the stability and the durable life of the element, R⁷ is more preferably a phenyl group or a tert-butylphenyl group. R⁸ is preferably a hydrogen atom or a phenyl group. R⁸ is more preferably a hydrogen atom from the viewpoint of easy availability of the compound.

In the general formula (1), R³ and R⁴ are each carbon atom having a substituent group. R³ and R⁴ may bond to each other; and R³ and R⁴ preferably bond to each other. In this case, the coordination property to a metal atom can be further enhanced by immobilizing the ring structure. Therefore, the driving voltage can be further reduced, and the luminous efficiency can be further improved. From the viewpoint of further enhancing the stability and the durable life of the light-emitting element, it is more preferable that R³ and R⁴ are bonded to each other with a vinylene group interposed therebetween, that is, in the general formula (1), the skeleton on the left side of L is a phenanthroline skeleton.

In the general formula (1), L is an unsubstituted arylene group having 6 to 20 carbon atoms. As described above, due to the fact that L is an unsubstituted arylene group having 6 to 20 carbon atoms, groups on both sides sandwiching L are easily conjugated. Therefore, the charge transporting property as a compound is further increased. In addition, since the number of carbon atoms of L is not too large, excessive increase in crystallinity is suppressed, and good film quality stability is provided. Therefore, the driving voltage in the case where the compound represented by the general formula (1) is used for a light-emitting element can be reduced, and the luminous efficiency and the durable life can be improved. From the viewpoint of further improving the luminous efficiency and the durable life, L is preferably a phenylene group, a naphthylene group, or a phenylenenaphthylene group.

The molecular weight of the compound represented by the general formula (1) is preferably 400 or more from the viewpoint of suppressing crystallization and improving the stability of film quality. On the other hand, from the viewpoint of improving the processability during sublimation purification and vapor deposition, the molecular weight of the compound represented by the general formula (1) is preferably 700 or less, and more preferably 640 or less.

Examples of the compound represented by the general formula (1) include the following compounds. The following are examples, and any compound other than the compounds specified herein is preferably used as long as it is represented by the general formula (1).

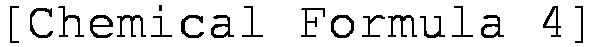

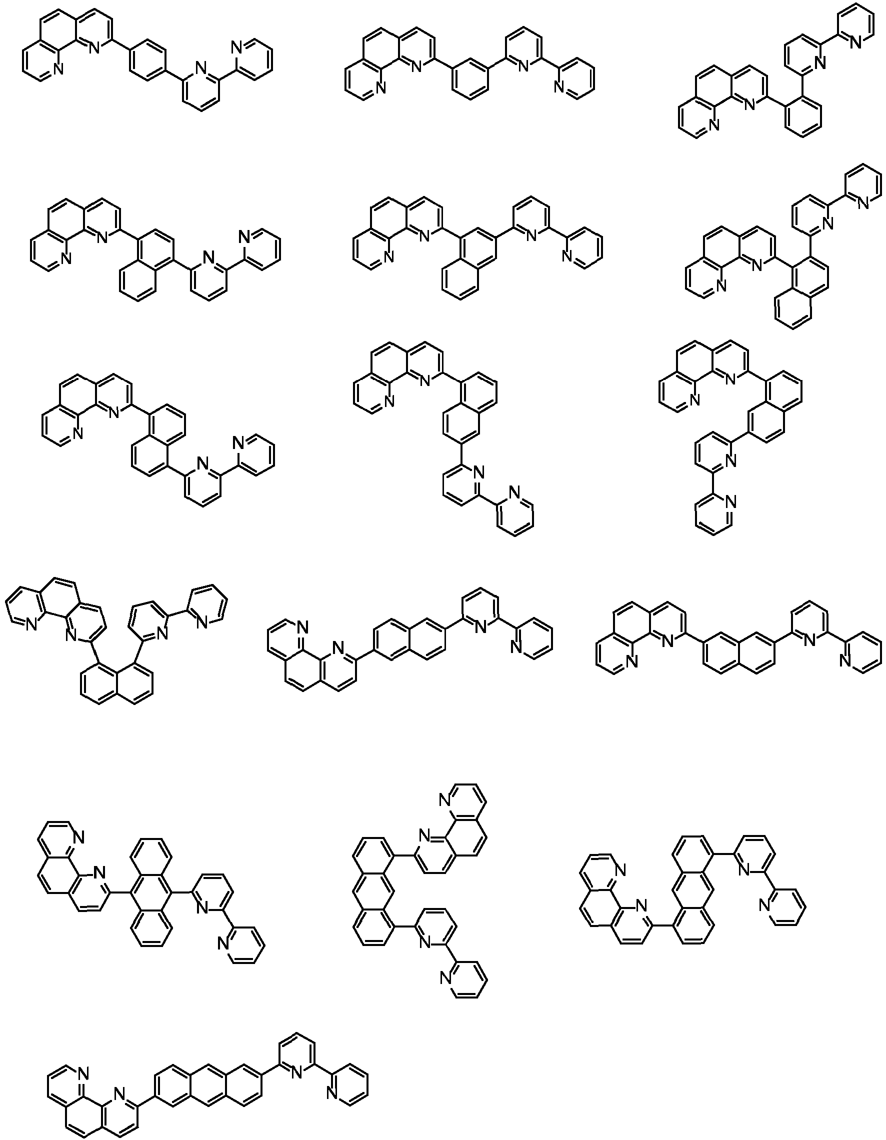

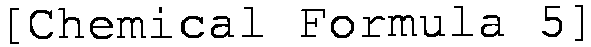

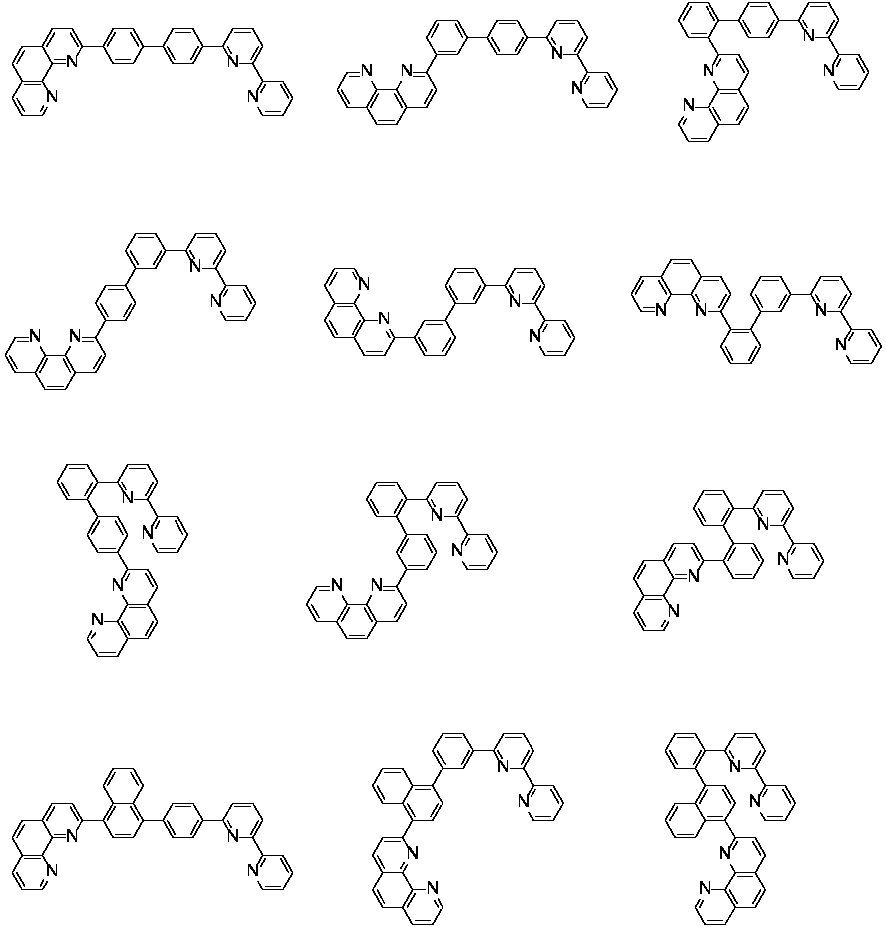

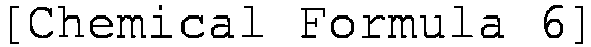

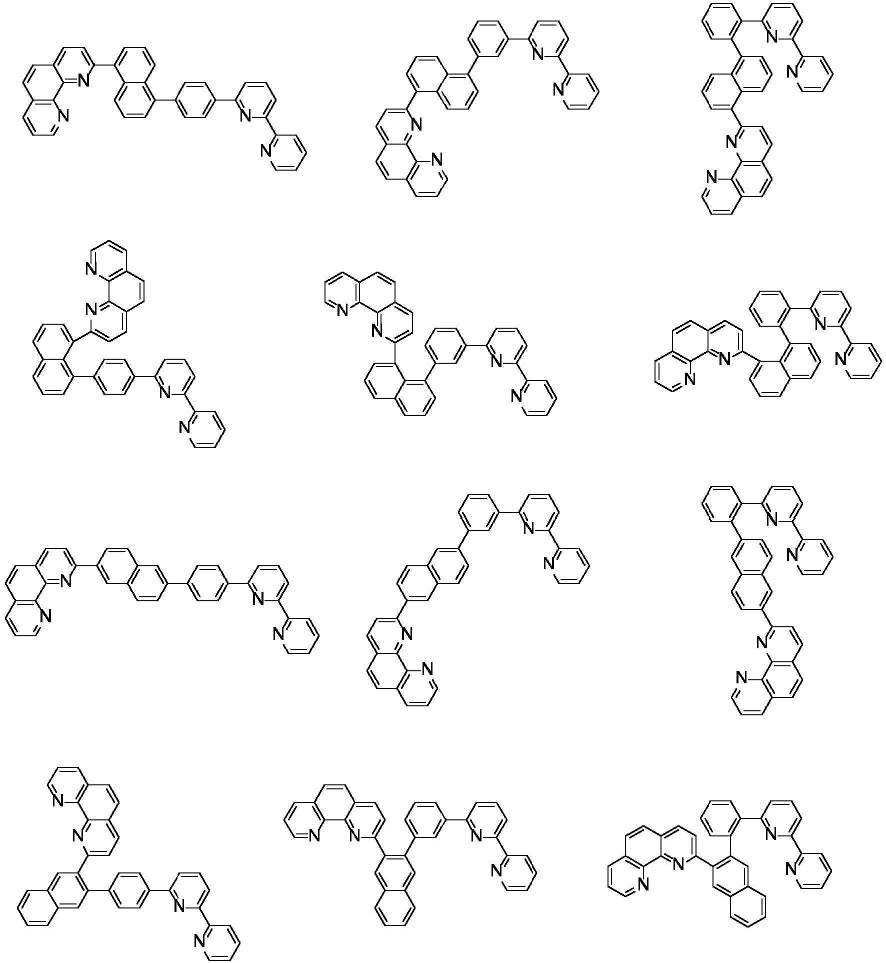

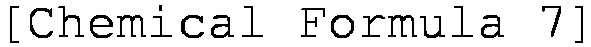

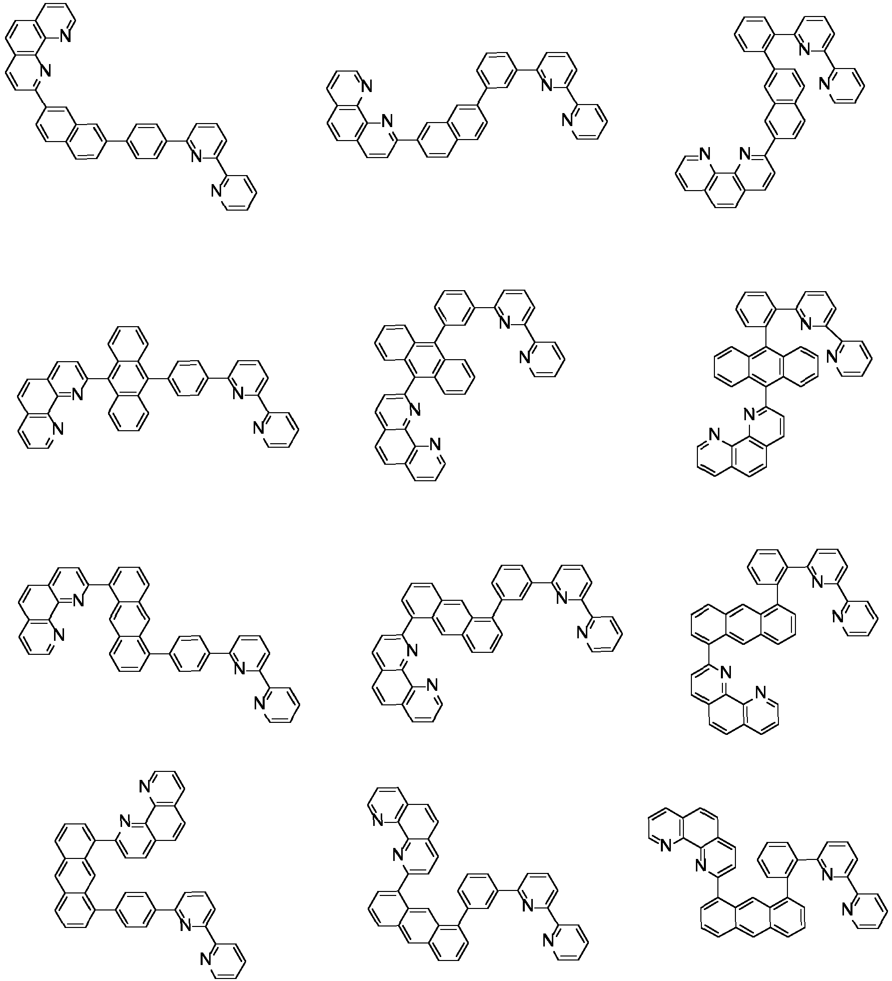

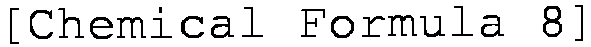

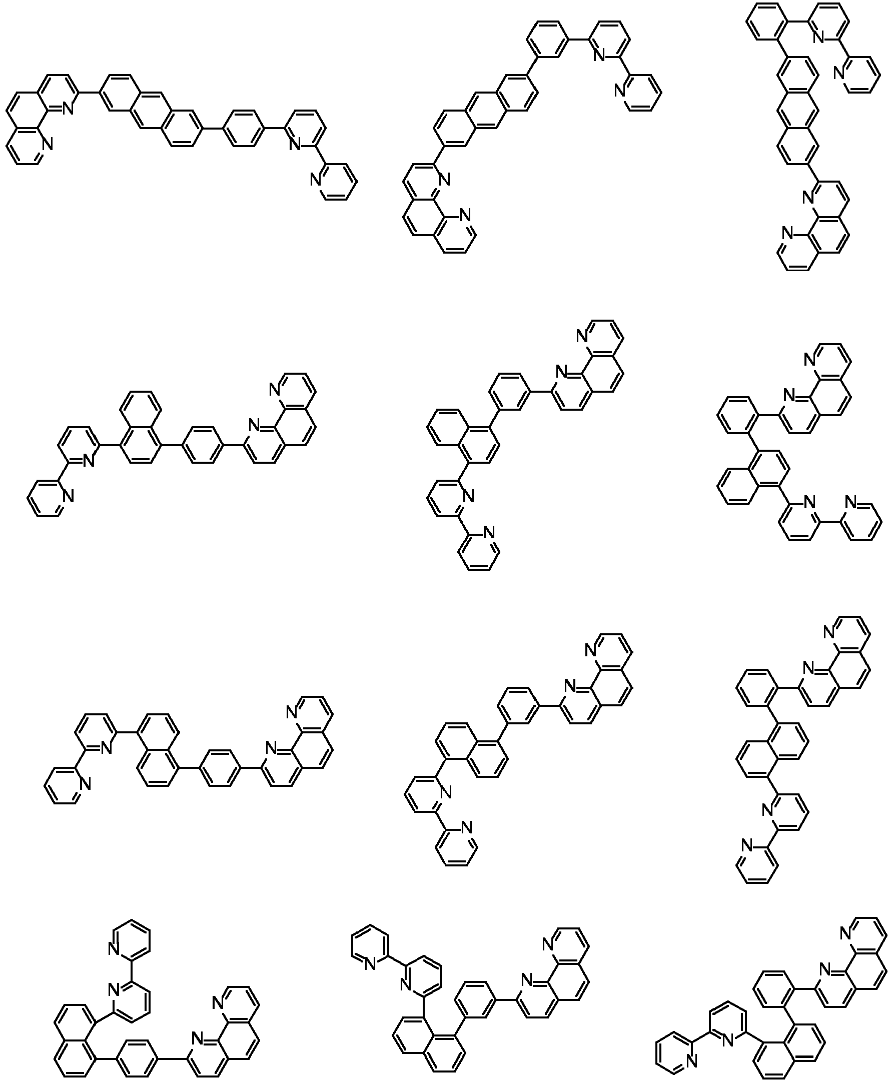

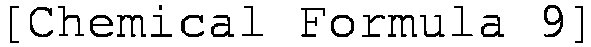

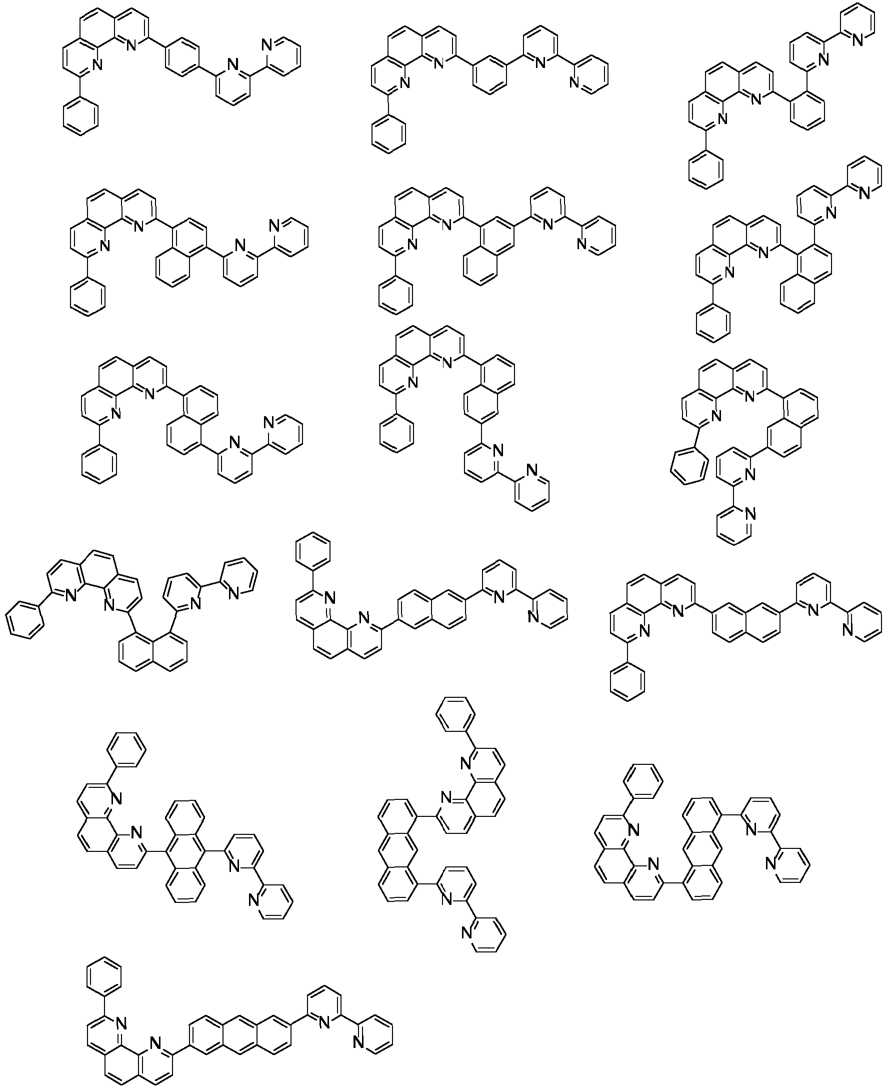

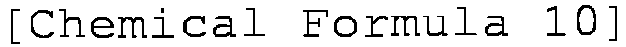

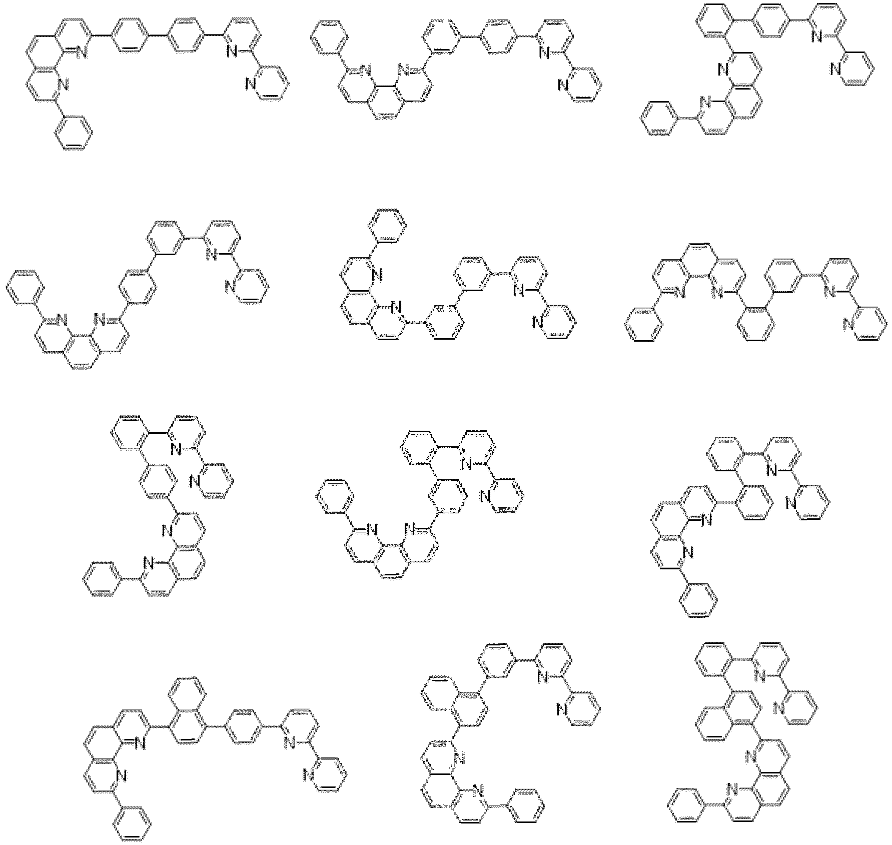

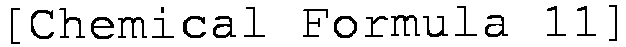

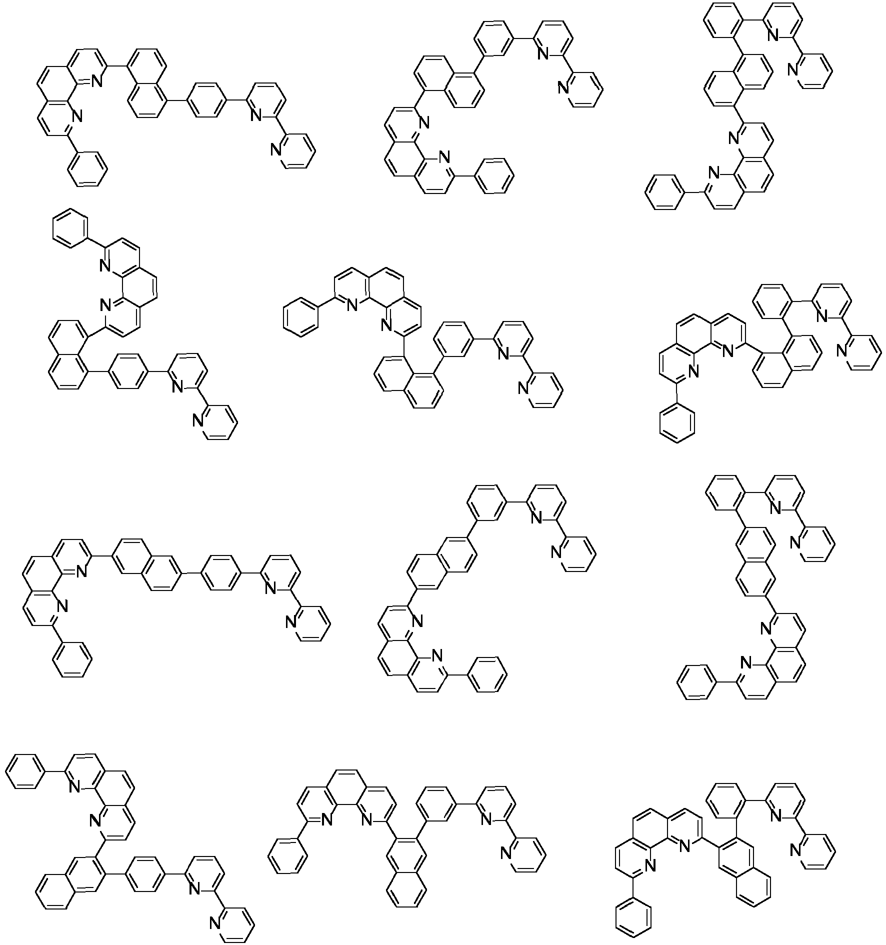

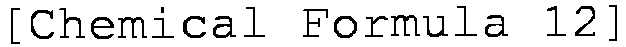

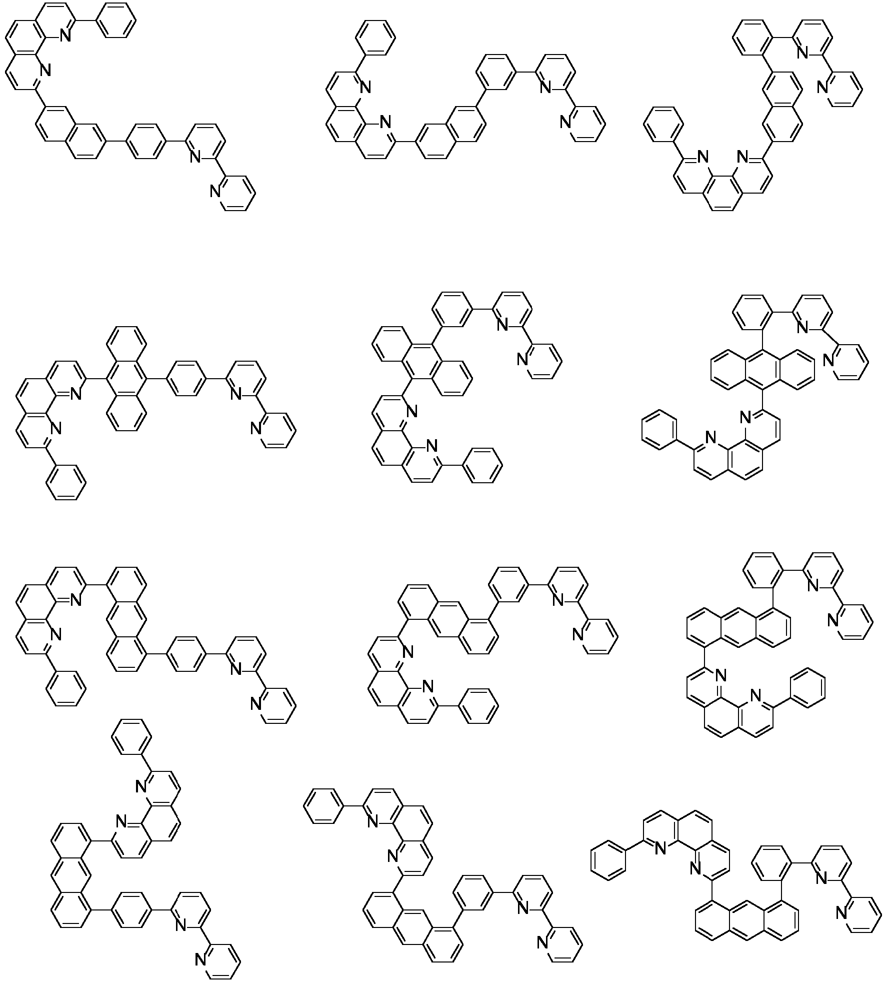

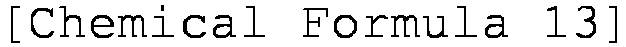

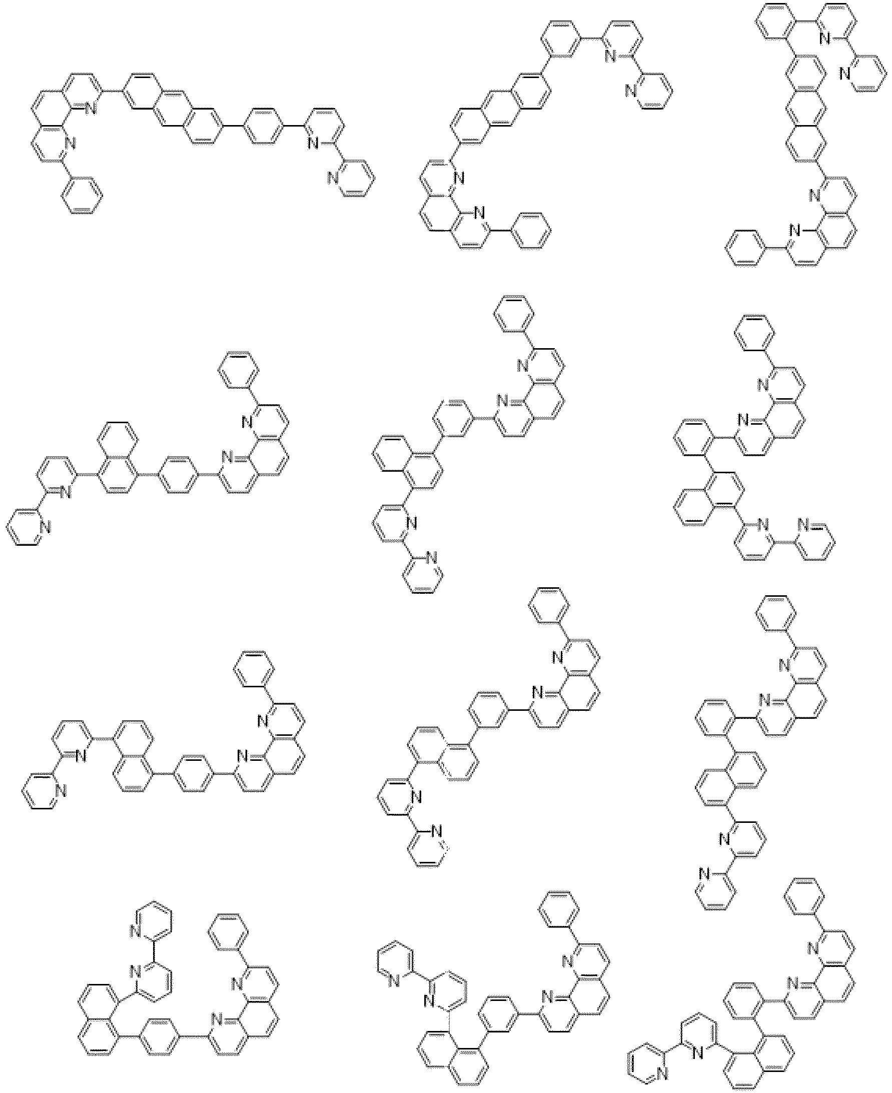

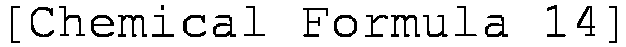

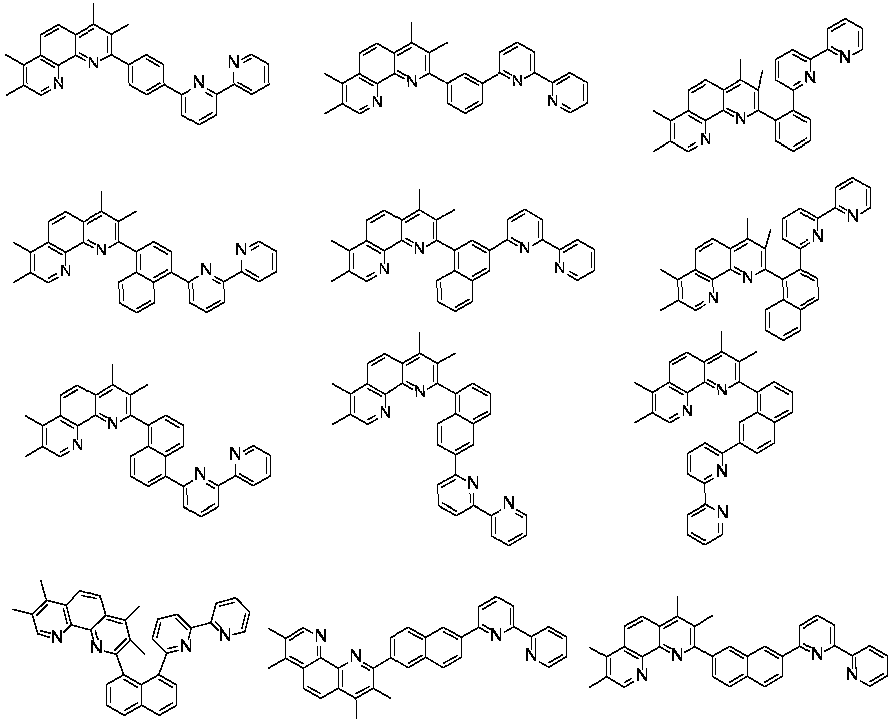

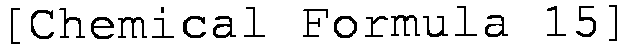

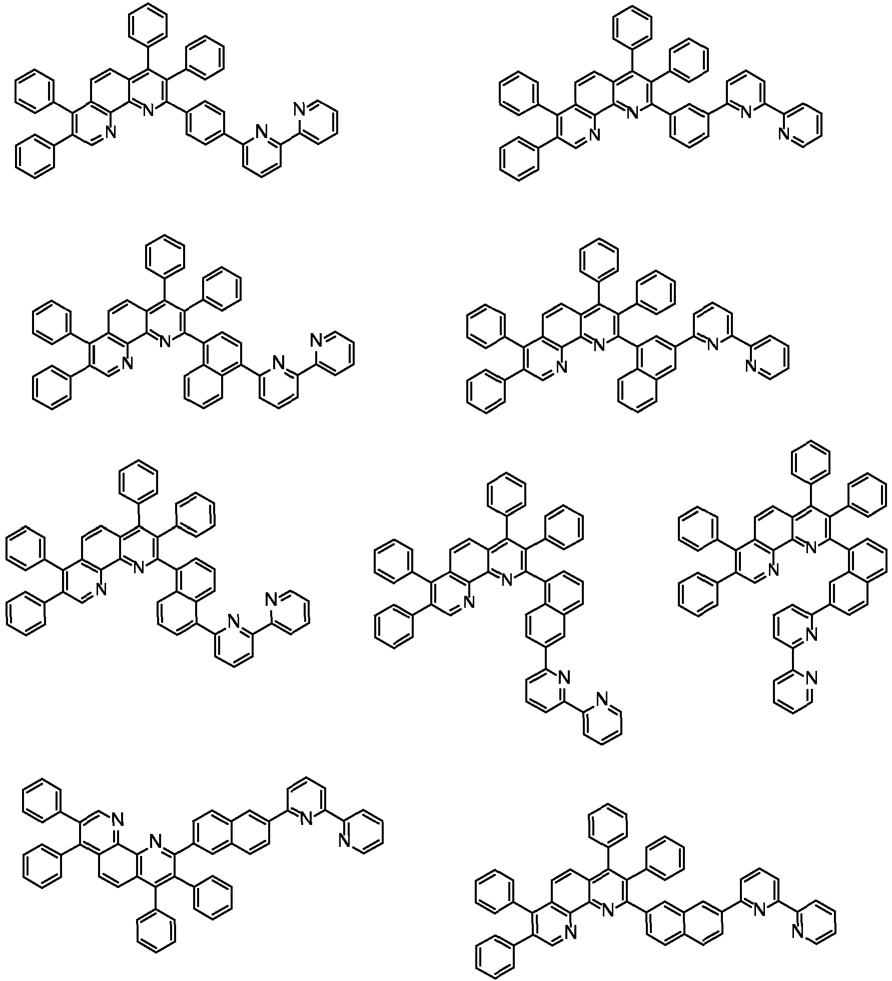

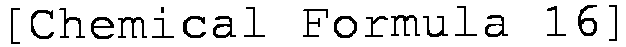

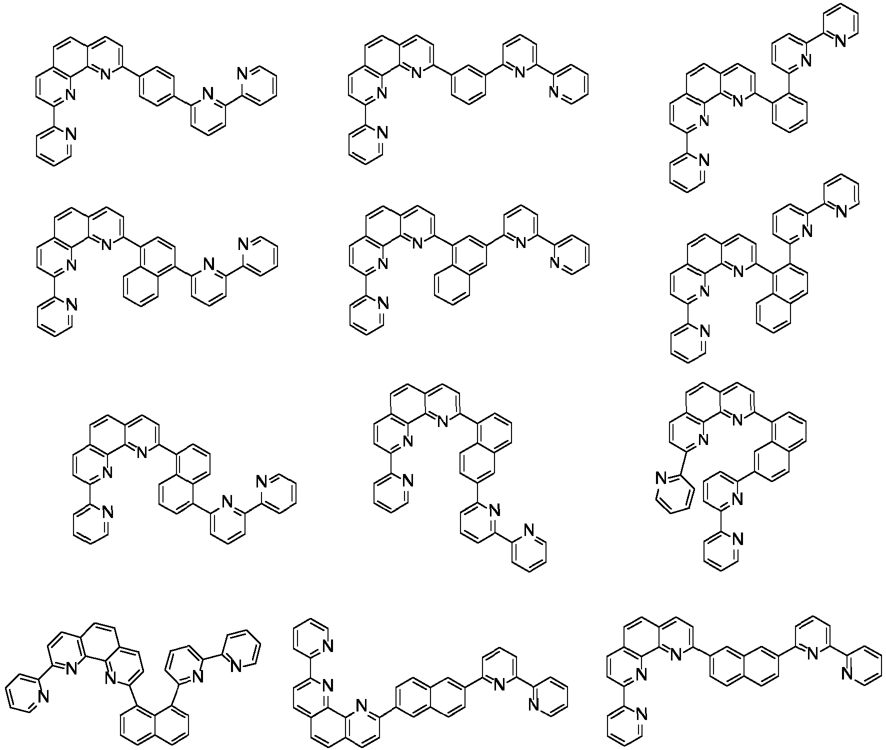

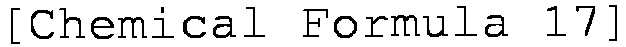

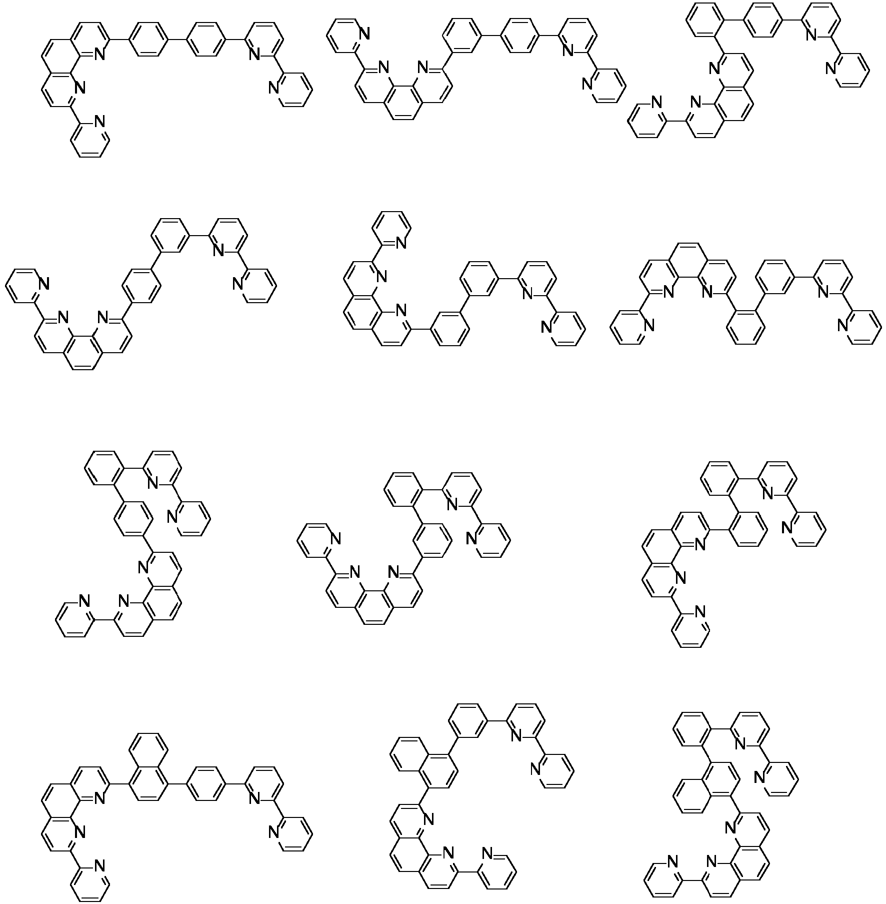

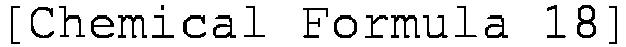

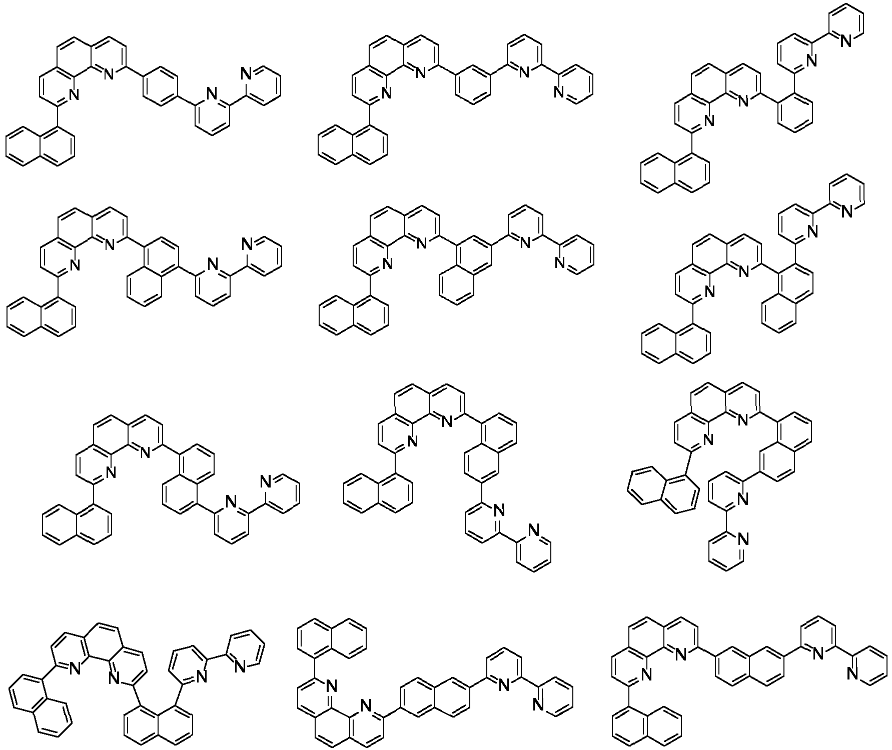

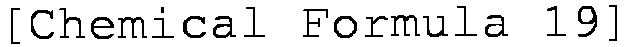

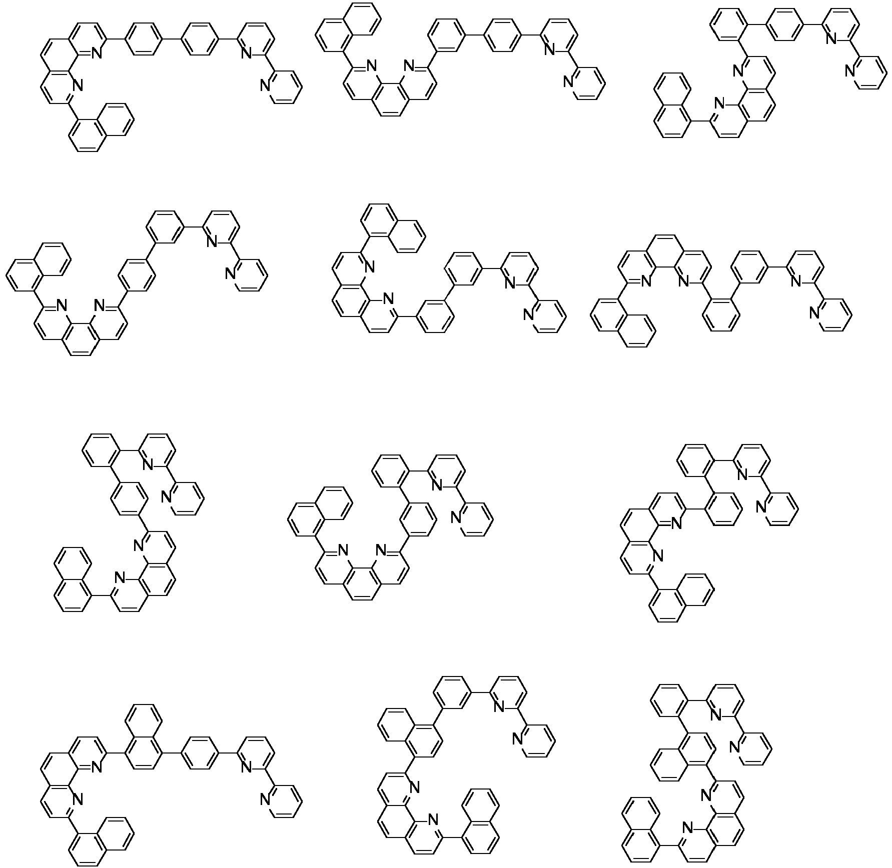

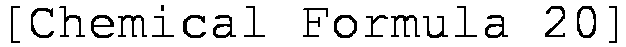

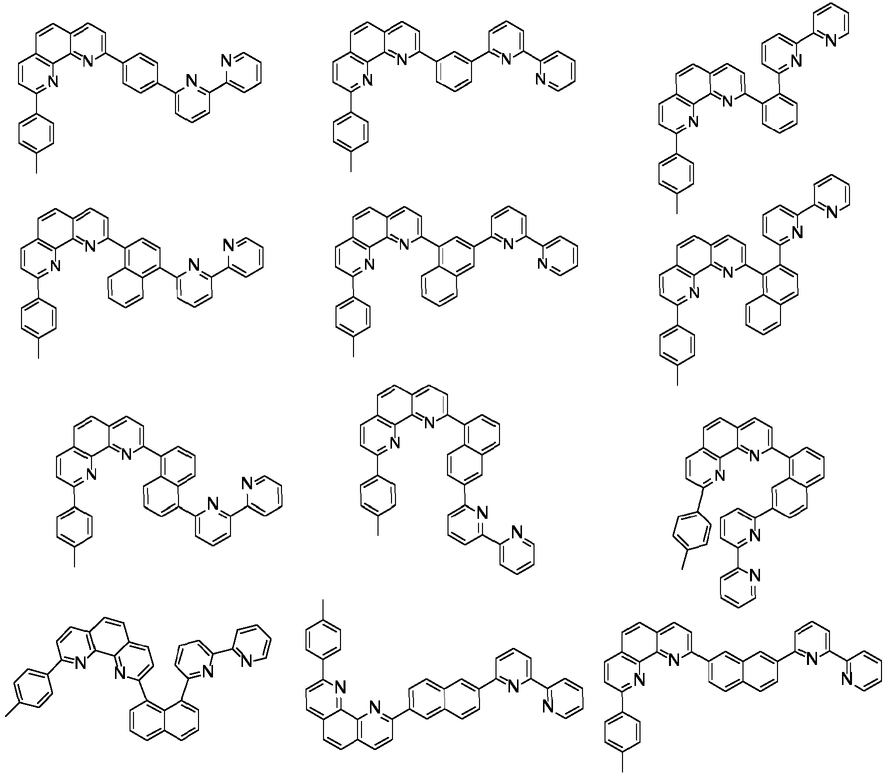

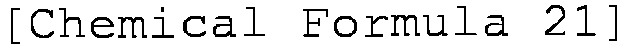

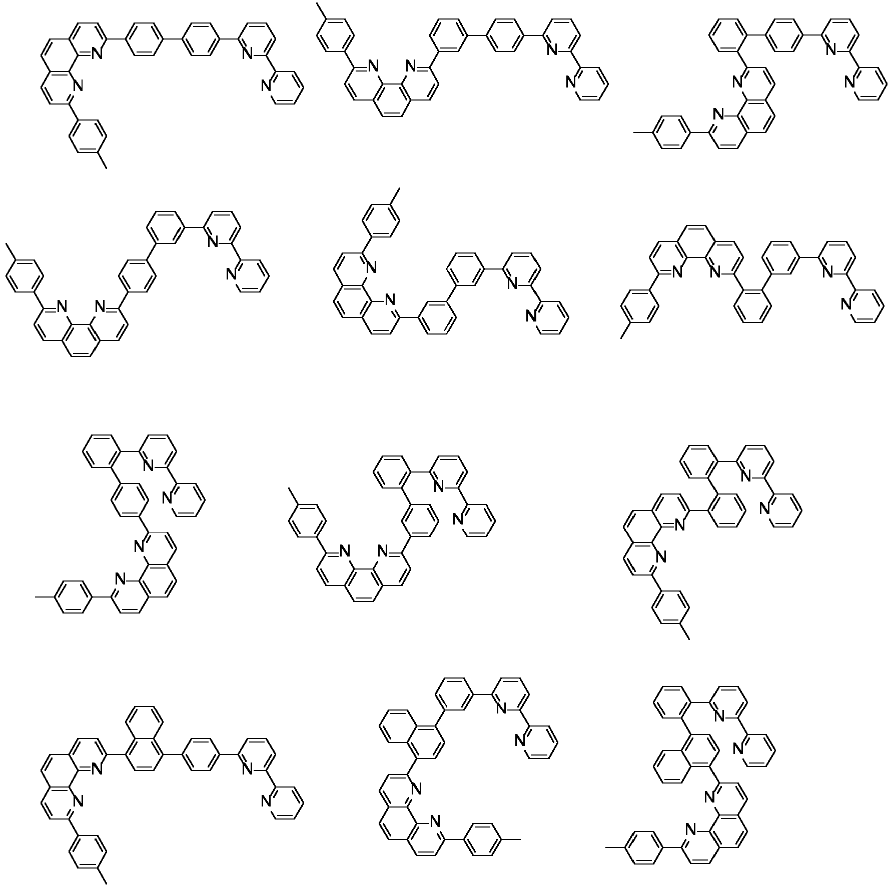

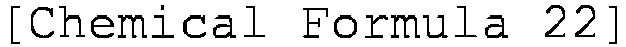

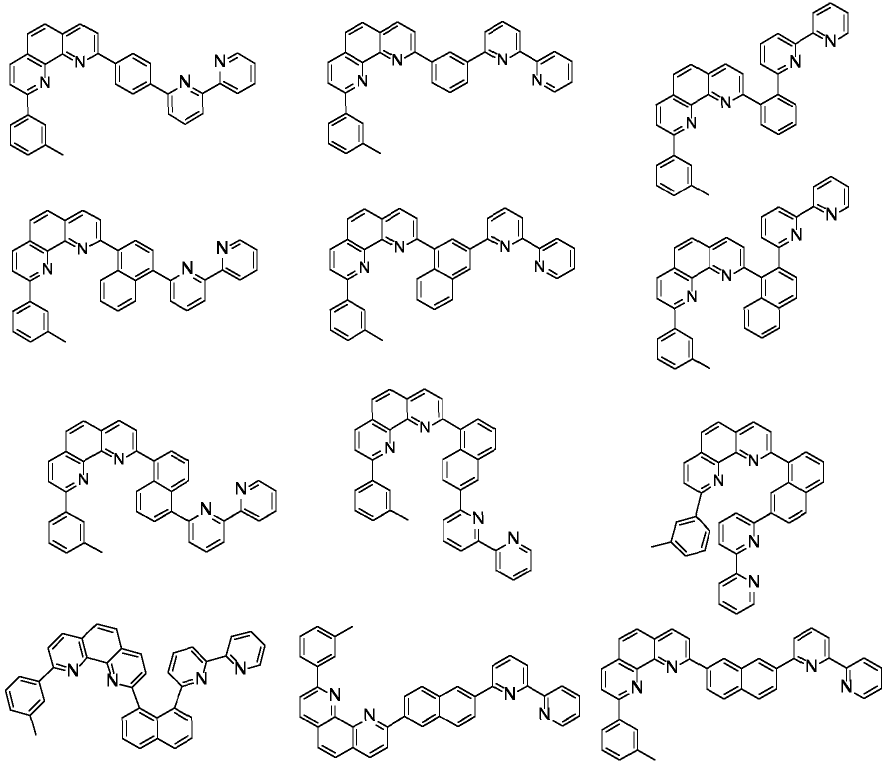

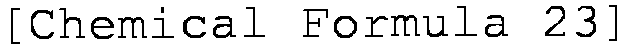

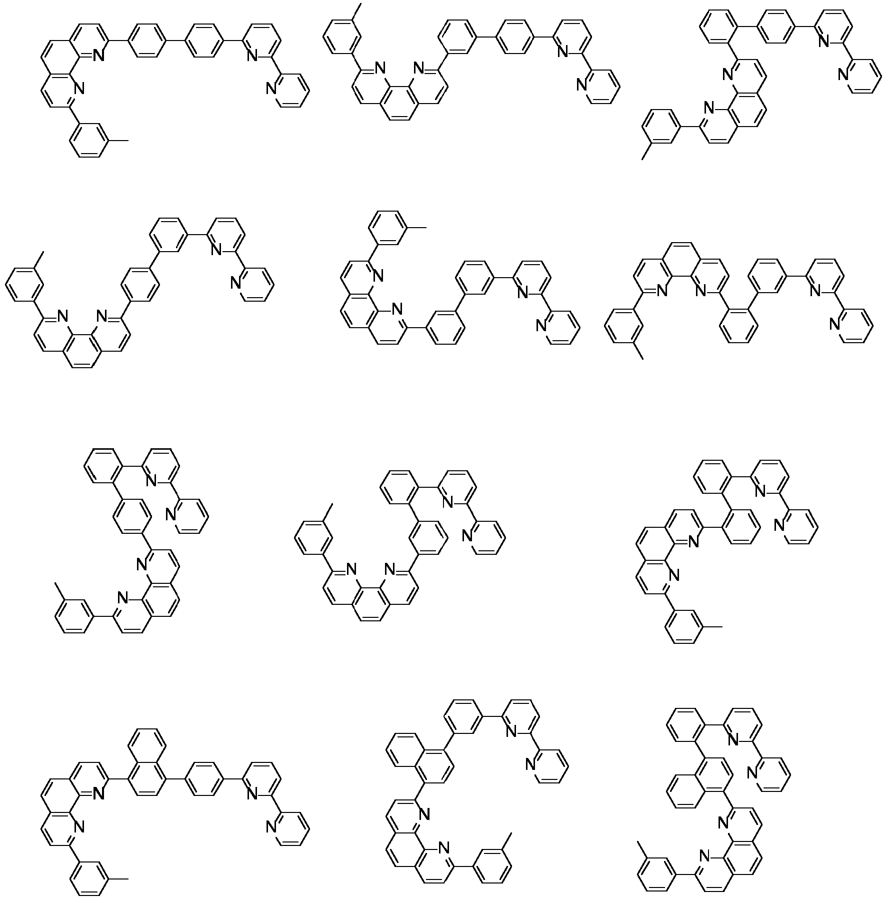

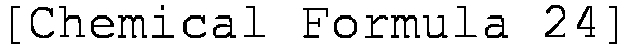

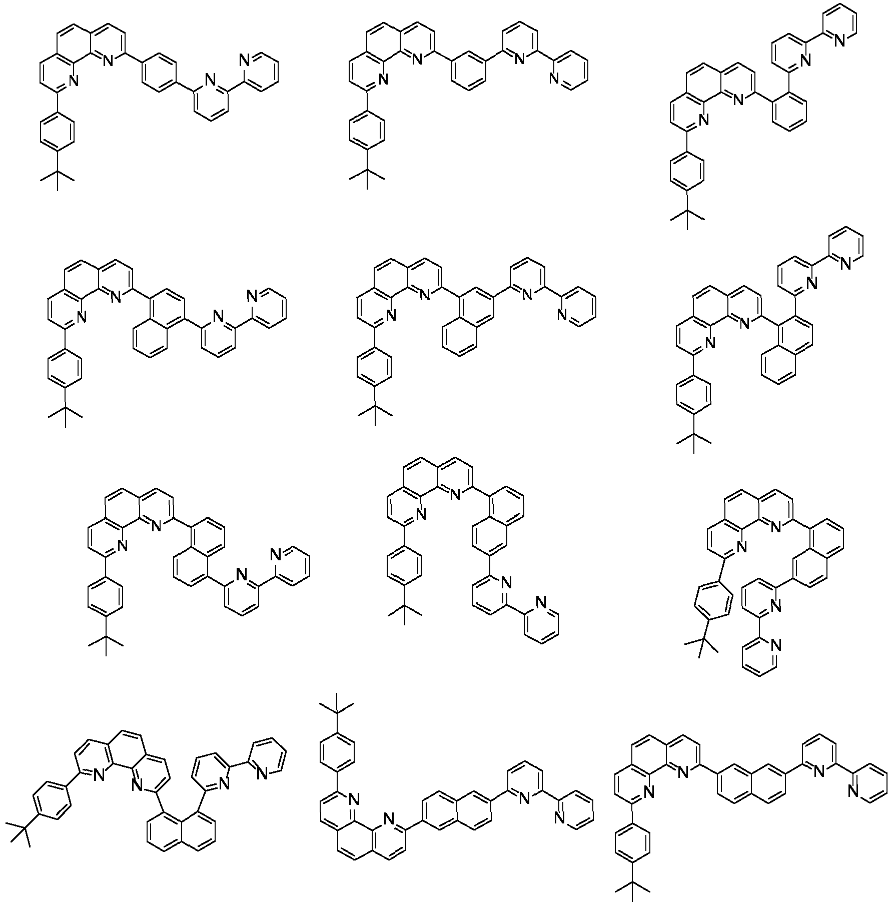

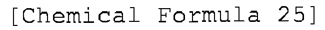

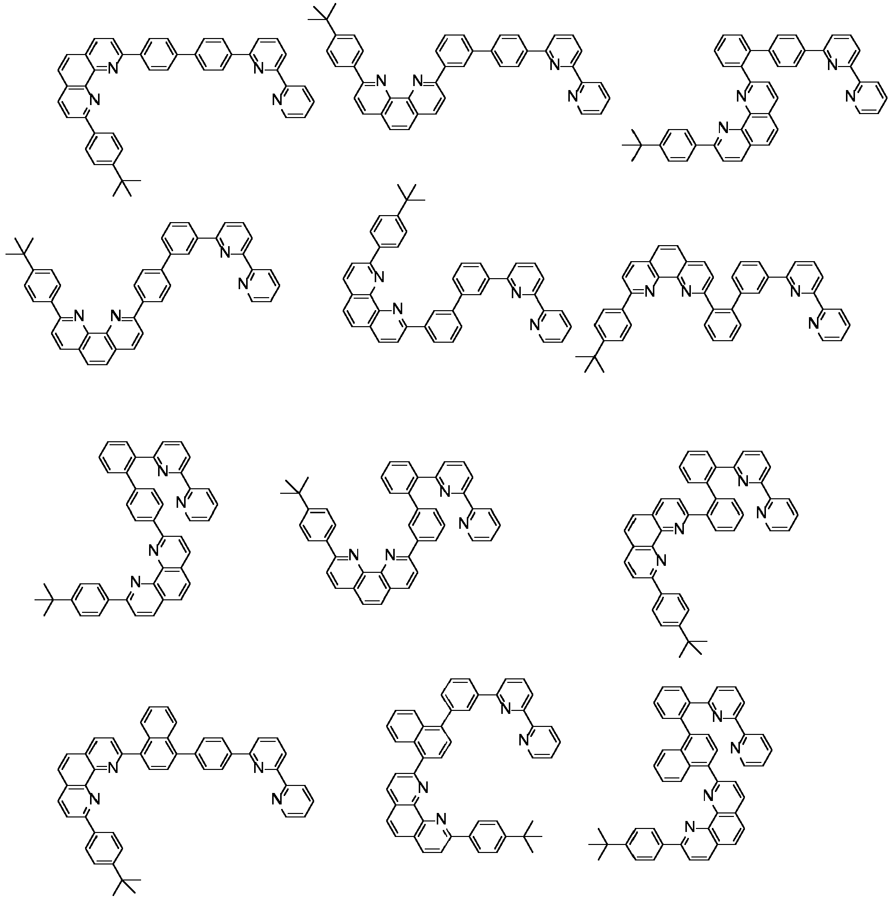

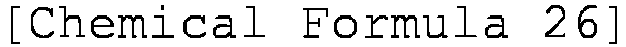

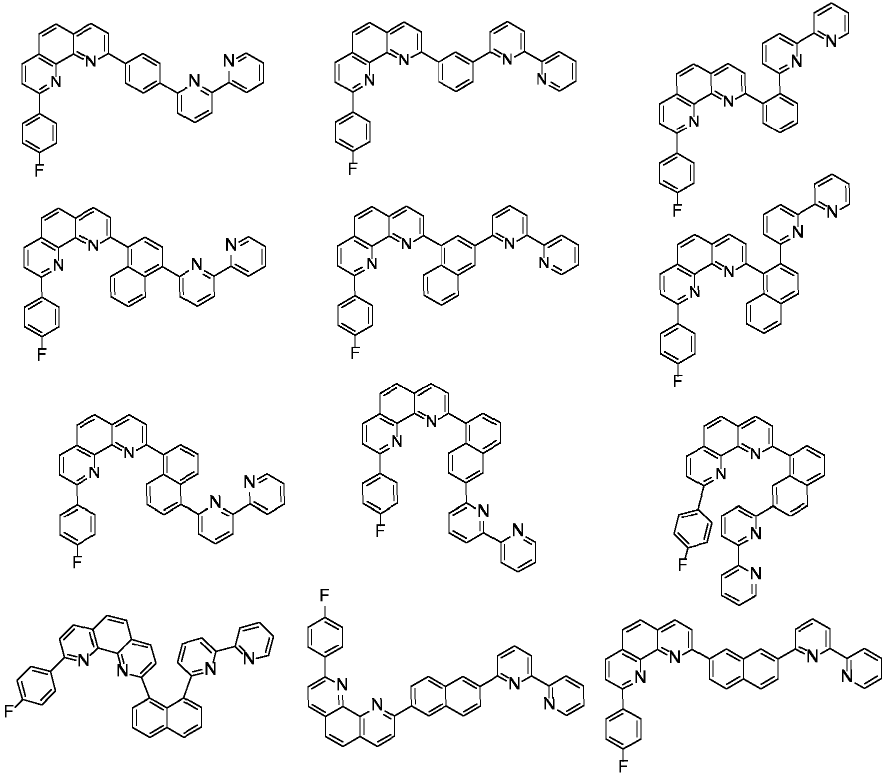

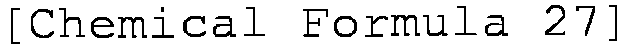

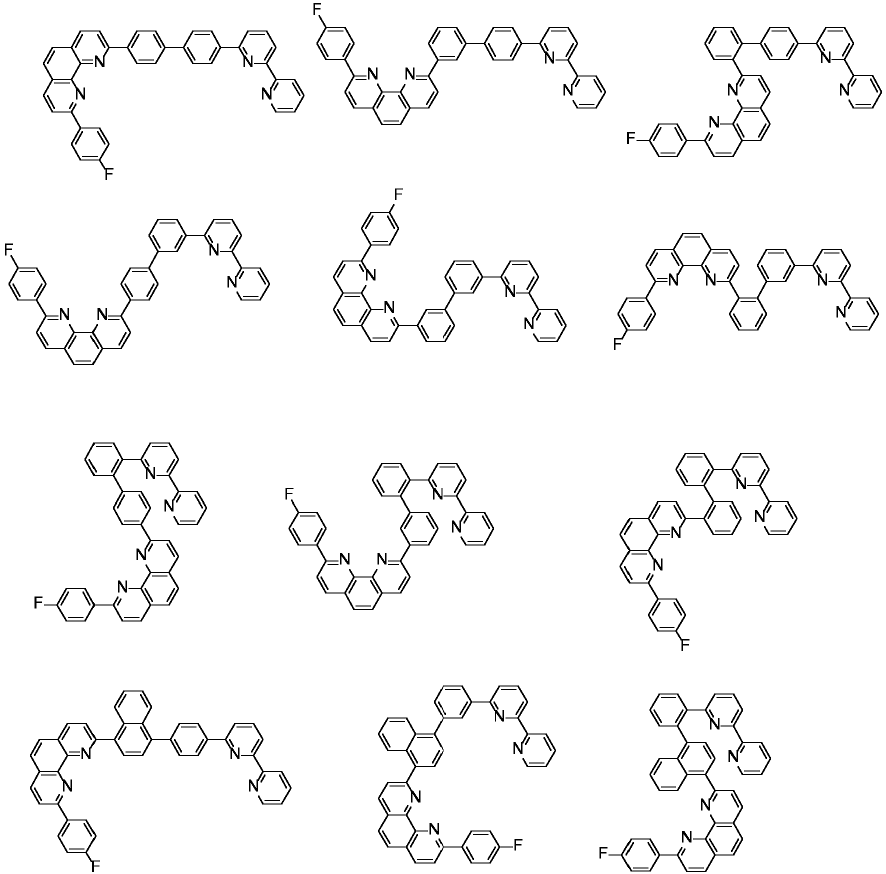

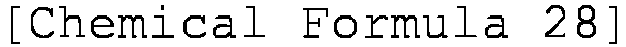

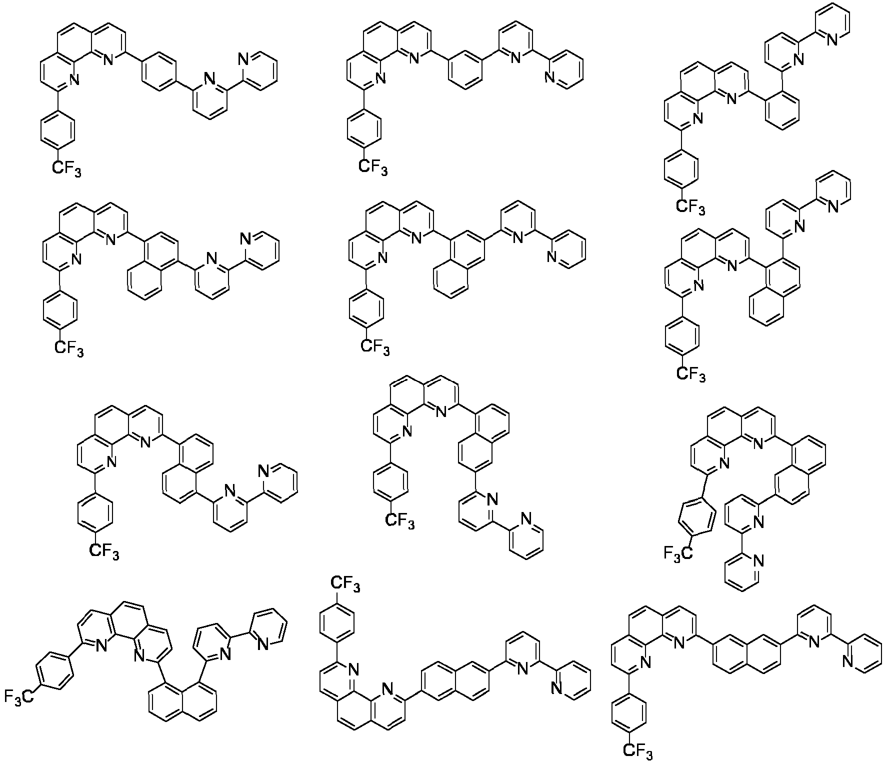

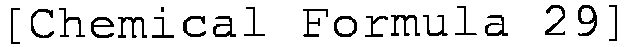

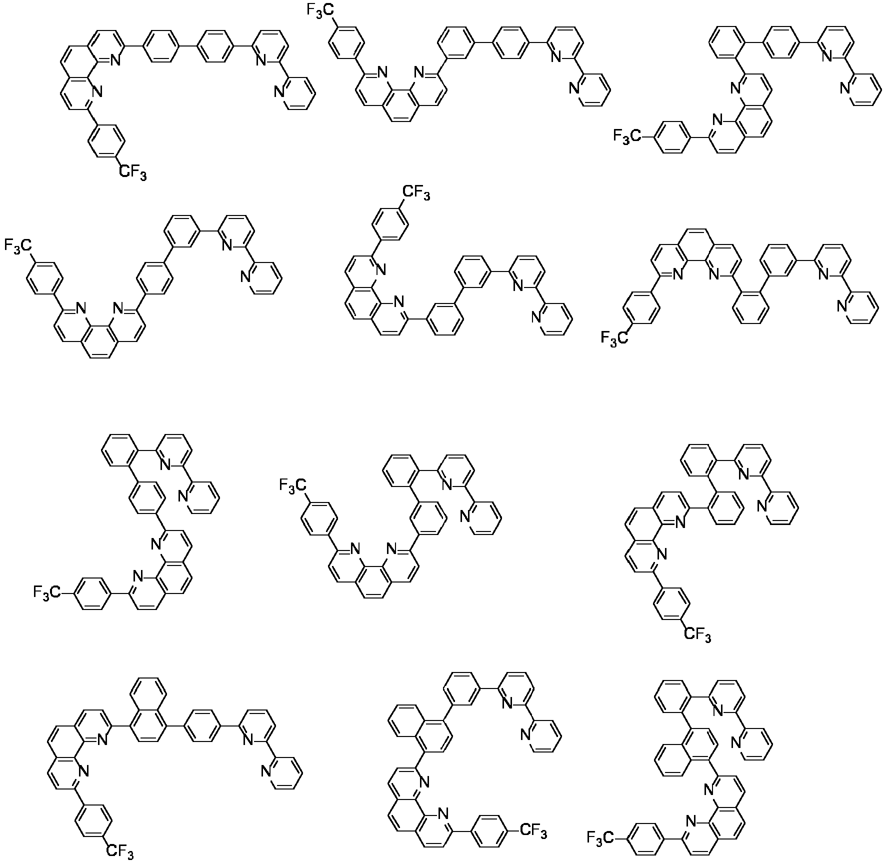

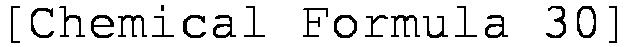

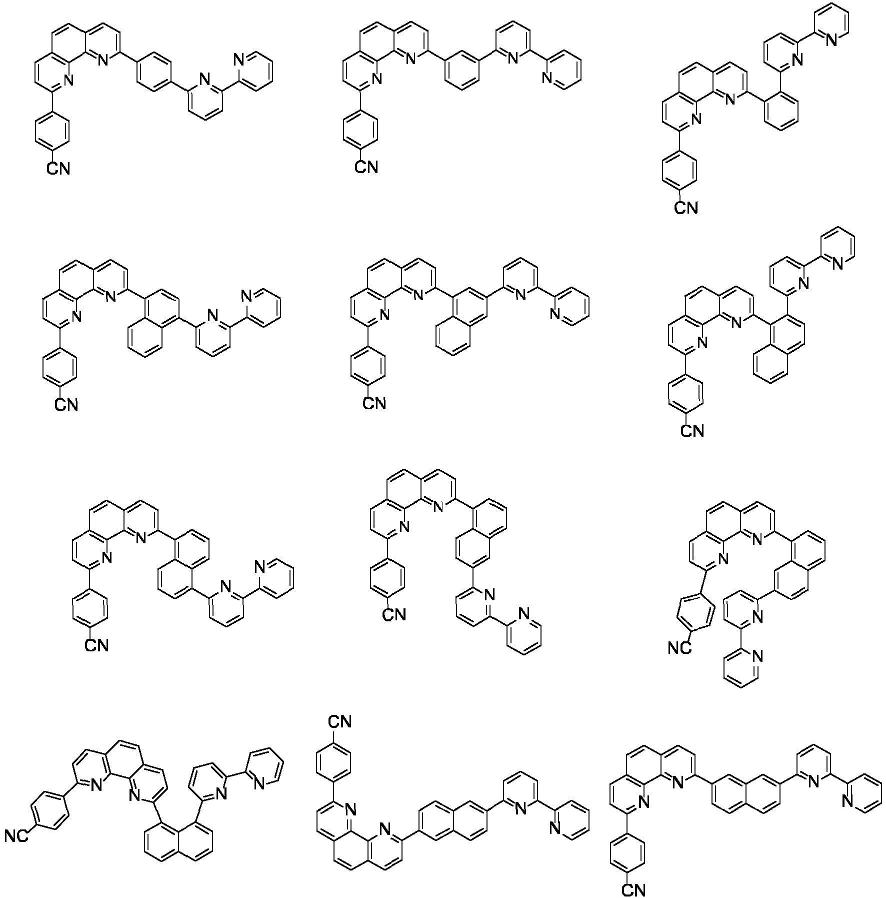

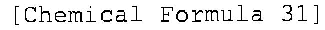

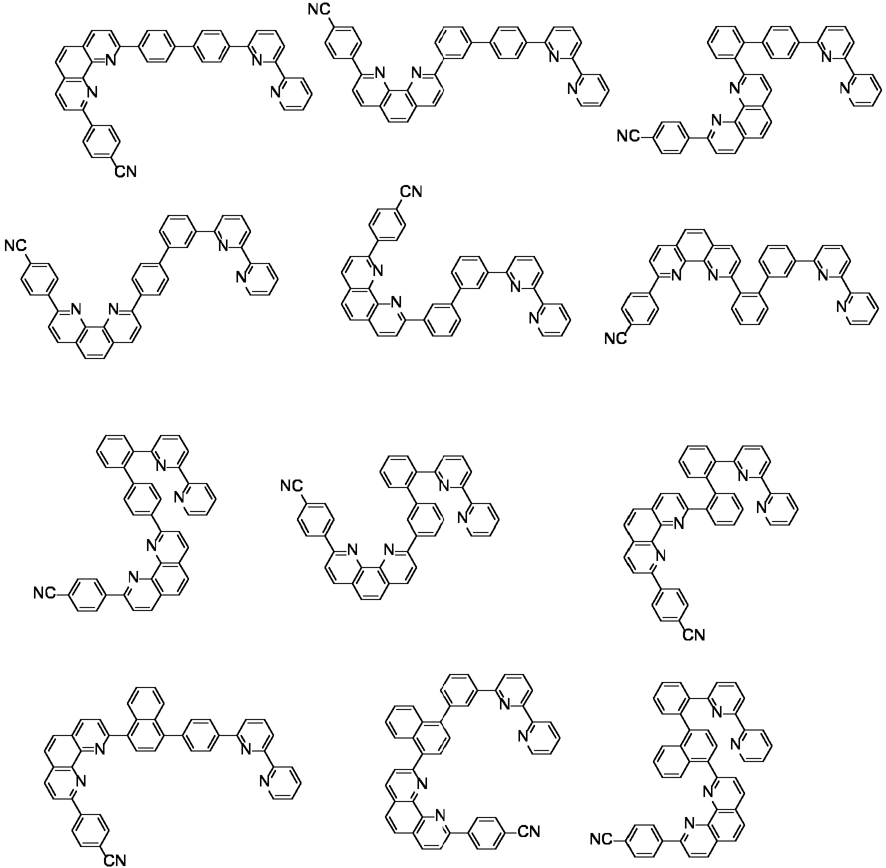

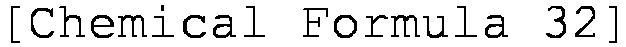

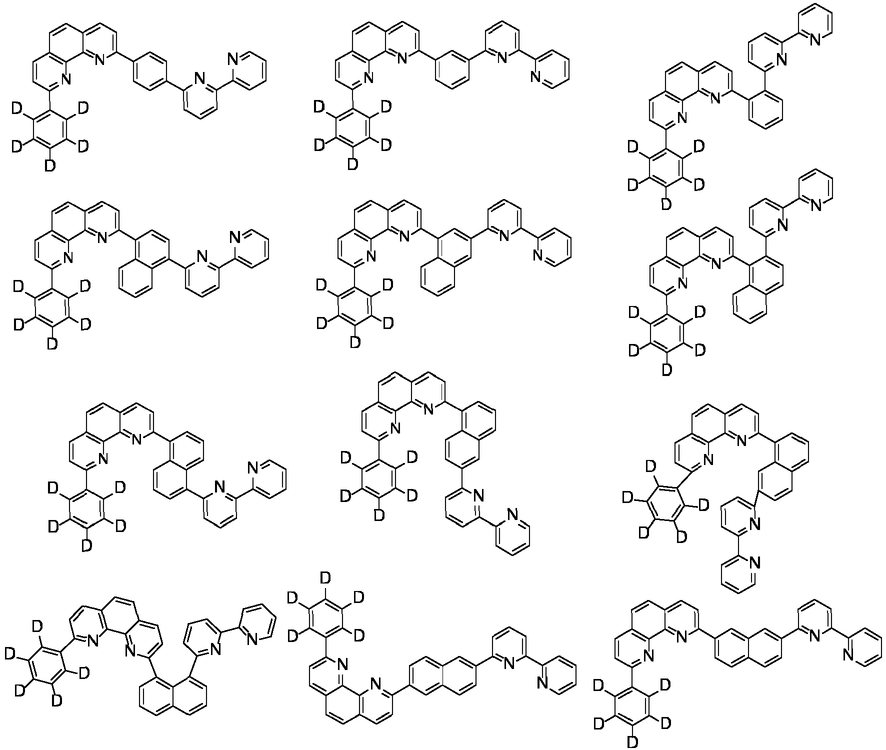

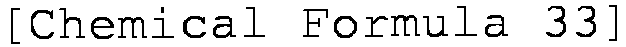

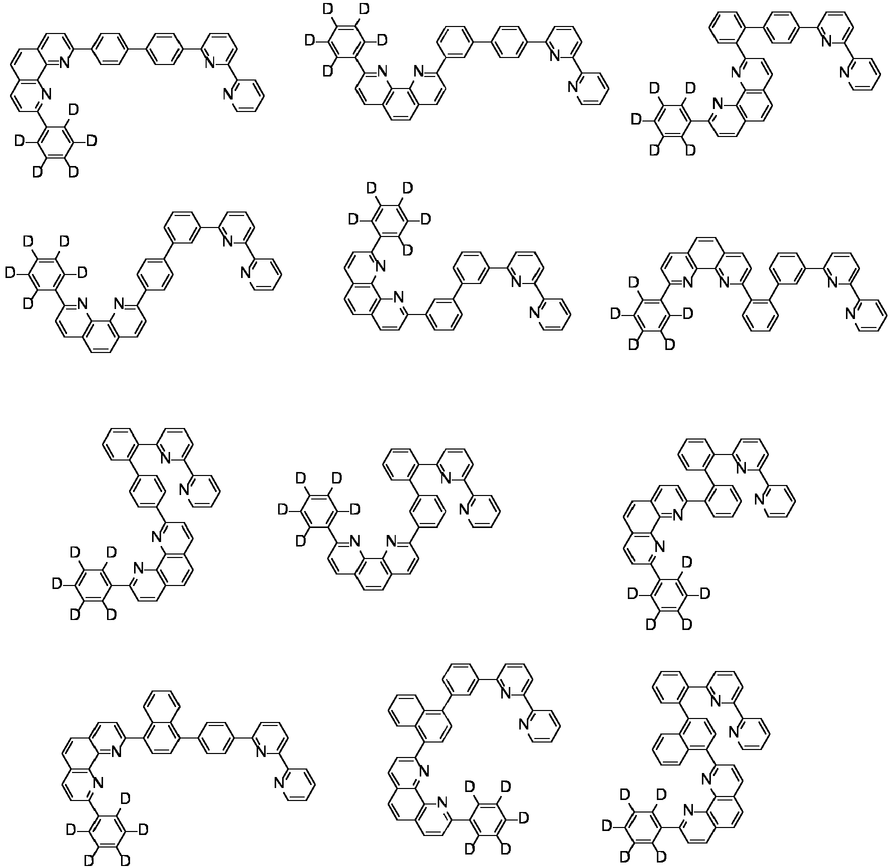

The compounds represented by the general formula (1) can be produced by a publicly known synthesis method. Examples of the synthesis method include, but are not limited to, a coupling reaction between a halogenated aryl derivative and an arylboronic acid derivative using palladium.

The compound represented by the general formula (1) is preferably used in any layer of the light-emitting element. As described later, the compound represented by the general formula (1) is suitably used for a hole injection layer, a hole transporting layer, an emissive layer, an electron transporting layer, a protective film (cap layer) of an electrode, and the like in a light-emitting element. By use of the material represented by the general formula (1) for any layer of a light-emitting element, a light-emitting element superior in luminous efficiency and durable life can be provided.

### (Light-Emitting Element)

The light-emitting element includes an anode, a cathode, and an organic layer interposed between the anode and the cathode, and the organic layer emits light by electrical energy. Such a light-emitting element may be referred to as an "organic EL element" in the following description.

Examples of the layer configuration between the anode and the cathode in the organic EL element include, other than the configuration of only an emissive layer, laminated configurations such as 1) an emissive layer/an electron transporting layer, 2) a hole transporting layer/an emissive layer, 3) a hole transporting layer/an emissive layer/an electron transporting layer, 4) a hole injection layer/a hole transporting layer/an emissive layer/an electron transporting layer, 5) a hole transporting layer/an emissive layer/an electron transporting layer/an electron injection layer, 6) a hole injection layer/a hole transporting layer/an emissive layer/an electron transporting layer/an electron injection layer, and 7) a hole injection layer/a hole transporting layer/an emissive layer/a hole inhibition layer/an electron transporting layer/an electron injection layer.

The light-emitting element may be a tandem type light-emitting element in which a plurality of the above-described layer configurations are laminated with an intermediate layer interposed therebetween. Generally, the intermediate layer is also called an intermediate electrode, an intermediate electroconductive layer, a charge generation layer, an electron draw-out layer, a connection layer, or an intermediate insulating layer, and for the intermediate layer, a known material configuration can be used. Specific examples of the tandem-type include laminated configurations which include a charge generation layer as an intermediate layer between an anode and a cathode, such as 8) a hole transporting layer/an emissive layer/an electron transporting layer/a charge generation layer/a hole transporting layer/an emissive layer/an electron transporting layer, and 9) a hole injection layer/a hole transporting layer/an emissive layer/an electron transporting layer/an electron injection layer/a charge generation layer/a hole injection layer/a hole transporting layer/an emissive layer/an electron transporting layer/an electron injection layer.

Each of the layers described above may be a single layer or a layer including a plurality of layers, and may be doped. In particular, the electron injection layer and the charge generation layer are preferably metal-doped layers doped with metal, and can improve electron transporting ability and electron injection ability to another layer adjacent thereto. In addition to the layers described above, a protective layer (cap layer) may be further included, and the luminous efficiency can be further improved by an optical interference effect.

The compound represented by the general formula (1) may be used for any of the layers described above in an organic EL element, but is particularly suitably used for an electron transporting layer, a charge generation layer, or an electron injection layer. Preferred examples of preferable configurations of the organic EL element according to an embodiment of the present invention include a configuration in which at least an electron transporting layer and an emissive layer are provided between the anode and the cathode, and the electron transporting layer contains a compound represented by the general formula (1), a configuration in which at least a charge generation layer and an emissive layer are provided between the anode and the cathode, and the charge generation layer contains a compound represented by the general formula (1), and a configuration in which at least an electron injection layer and an emissive layer are provided between the anode and the cathode, and the electron injection layer contains a compound represented by the general formula (1).

In the organic EL element according to an embodiment of the present invention, the anode and the cathode have a role of supplying a sufficient current for light emission of the element, and at least one of the anode and the cathode is desirably transparent or translucent for light extraction. Usually, the anode formed on a substrate is made to be a transparent electrode.

### (Substrate)

The organic EL element is preferably formed on a substrate to maintain the mechanical strength of the organic EL element. Examples of the substrate include a glass substrate such as a soda glass substrate or an alkali-free glass substrate, and a plastic substrate. The thickness of the glass substrate is just required to be a thickness sufficient for maintaining mechanical strength and a thickness of 0.5 mm or more is large enough. Regarding the material of glass, it is preferred that the amount of ions eluted from the glass is small, and alkali-free glass is preferable. Soda lime glass barrier-coated with SiO₂ or the like is commercially available, and can also be used.

### (Anode)

It is preferable that the material to be used for the anode can efficiently inject holes into the organic layer. It is preferable for extraction of light that the material to be used for the anode is transparent or translucent. Examples of the material to be used for the anode include electroconductive metal oxides such as zinc oxide, tin oxide, indium oxide, indium tin oxide (ITO), and indium zinc oxide (IZO), metals such as gold, silver, and chromium, inorganic electroconductive substances such as copper iodide and copper sulfide, or electroconductive polymers such as polythiophene, polypyrrole, and polyaniline. Among them, ITO glass and NESA glass are preferable. These electrode materials may be used singly, or two or more of them may be laminated on or mixed with each other upon use.

### (Cathode)

The material to be used for the cathode is not particularly limited as long as the material can efficiently inject electrons into the emissive layer. Examples of the material to be used for the cathode include metals such as platinum, gold, silver, copper, iron, tin, aluminum, and indium, or alloys or multilayer laminate of these metals with metals having a low work function such as lithium, sodium, potassium, calcium and magnesium. Among them, from the viewpoint of electric resistance value, ease of film formation, stability of film, luminous efficiency, etc., aluminum, silver, and magnesium are preferable as a main component, and it is more preferable that the material to be used for the cathode is composed of magnesium and silver because injection of electrons into the electron transporting layer and the electron injection layer is easy.

### (Protective Layer)

To protect the cathode, the cathode is preferably laminated with a protective layer (cap layer). The material (capping material) to form the protective layer is not particularly limited, and examples of the material include metals such as platinum, gold, silver, copper, iron, tin, aluminum, and indium, alloys in which such a metal is used, inorganic substances such as silica, titania, and silicon nitride, and organic polymer compounds such as polyvinyl alcohol, polyvinyl chloride, and hydrocarbon-based polymer compounds. The compound represented by the general formula (1) can also be used as a capping material. It is noted that when the organic EL element has an element structure in which light is extracted from the cathode side (top emission structure), the capping material preferably has a light transmitting property in a visible light region.

### (Hole Injection Layer)

The hole injection layer is a layer to be interposed between the anode and the hole transporting layer. The hole injection layer may be a single layer or a laminate in which a plurality of layers are laminated. The fact that the hole injection layer is present between the hole transporting layer and the anode is preferable because owing to this fact, not only the driving voltage is further reduced and the durable life is improved, but also the carrier balance of the element is improved to improve the luminous efficiency.

As the material to be used for the hole injection layer, a publicly known material can be used. Examples of such a material include benzidine derivatives, materials called starburst arylamines, triarylamine derivatives, heterocyclic compounds such as biscarbazole derivatives, pyrazoline derivatives, stilbene-based compounds, fluorene-based compounds, hydrazone-based compounds, benzofuran derivatives, thiophene derivatives, oxadiazole derivatives, phthalocyanine derivatives, and porphyrin derivatives, and polymer-based materials such as polycarbonates and styrene derivatives having the above-described monomer in a side chain, polythiophene, polyaniline, polyfluorene, polyvinylcarbazole, and polysilane. Benzidine derivatives, starburst arylamine-based materials, and fluorene-based compounds are more preferably used from the viewpoint of smoothly injecting and transporting holes from the anode to the hole transporting layer.

These materials may be used alone, or two or more materials may be mixed and used. A plurality of materials may be stacked to form a hole injection layer. Furthermore, it is more preferable that the hole injection layer is formed of an acceptor compound alone or a hole injection material as described above is doped with an acceptor compound and then used because the above-described effects can be obtained more remarkably. The acceptor compound is a material that forms a charge transfer complex together with a hole transporting layer in contact therewith when the acceptor compound is used as a single layer film, or together with a material forming a hole injection layer when the acceptor compound is used as a dopant. Use of such a material leads to improvement in the electroconductivity of the hole injection layer, further contributes to reduction in the driving voltage of the element, and can further improve the luminous efficiency and the durable life.

As the acceptor compound, a publicly known material can be used. Examples thereof include metal chlorides, metal oxides such as molybdenum oxide, charge transfer complexes, organic compounds having a nitro group, a cyano group, a halogen or a trifluoromethyl group in the molecule, quinone-based compounds, acid anhydride-based compounds, and fullerenes. Among these compounds, metal oxides and cyano group-containing compounds are preferable because metal oxides and cyano group-containing compounds are easily handled and vapor-deposited, and therefore the above-described effects are easily obtained. In either of the case in which the hole injection layer is formed of an acceptor compound alone or the case in which the hole injection layer is doped with an acceptor compound, the hole injection layer may be a single layer or may include a plurality of layers that are laminated.

### (Hole Transporting Layer)

The hole transporting layer is a layer that transports holes injected from the anode to the emissive layer. The hole transporting layer may be a single layer or may include a plurality of layers that are laminated.

Examples of the material to be used for the hole transporting layer include those enumerated as examples of the material to be used for the hole injection layer. From the viewpoint of smoothly injecting and transporting holes into the emissive layer, triarylamine derivatives and benzidine derivatives are more preferable.

### (Emissive Layer)

The emissive layer may be either a single layer or a plurality of layers. Each emissive layer is formed of a light-emitting material (a host material and a dopant material), which may be a mixture of a host material and a dopant material, or a host material alone, or a mixture of two host materials and a single dopant material. That is, in the organic EL element in an embodiment of the present invention, in each emissive layer, only a host material or a dopant material may emit light, or both a host material and a dopant material may emit light. From the viewpoint of efficiently utilizing the electrical energy and obtaining light emission of high color purity, the emissive layer is preferably made from a mixture of a host material and a dopant material. Further, each of the host material and the dopant material may be of one type or a combination of multiple types. The dopant material may be contained in the entire host material or may be partially contained therein. The dopant material may be either laminated or dispersed. The dopant material can control an emitted color. From the viewpoint of controlling a concentration quenching phenomenon, the amount of the dopant material is preferably 30% by weight or less, and more preferably 20% by weight or less based on the host material. In a doping process, the dopant material can be applied by a method of co-deposition with a host material. Alternatively, the dopant material may be preliminarily mixed a host material and vapor-deposited simultaneously with the host material.

As the light-emitting material, publicly known materials can be used. Examples thereof include fused ring derivatives such as anthracene and pyrene, which are known as luminous bodies, metal chelated oxynoid compounds such as tris(8-quinolinolate)aluminum, bisstyryl derivatives such as bisstyrylanthracene derivatives and distyrylbenzene derivatives, tetraphenyl butadiene derivatives, indene derivatives, coumarin derivatives, oxadiazole derivatives, pyrrolopyridine derivatives, perinone derivatives, cyclopentadiene derivatives, oxadiazole derivatives, thiadiazolopyridine derivatives, dibenzofuran derivatives, carbazole derivatives, indolocarbazole derivatives, and polymers such as polyphenylenevinylene derivatives, polyparaphenylene derivatives, and polythiophene derivatives.

The host material contained in the light-emitting material is not limited to only one compound, and a mixture of multiple compounds may be used. Alternatively, the host materials may be laminated and used. As the host material, publicly known materials can be used. The host material is not particularly limited, and examples thereof include compounds having a fused aryl ring such as naphthalene, anthracene, phenanthrene, pyrene, chrysene, naphthacene, triphenylene, perylene, fluoranthene, fluorene, and indene, and derivatives thereof, aromatic amine derivatives such as N,N'-dinaphthyl-N,N'-diphenyl-4,4'-diphenyl-1,1'-diamine, metal chelated oxynoid compounds such as tris(8-quinolinato)aluminum(III), bisstyryl derivatives such as distyrylbenzene derivatives, tetraphenylbutadiene derivatives, indene derivatives, coumarin derivatives, oxadiazole derivatives, pyrrolopyridine derivatives, perinone derivatives, cyclopentadiene derivatives, pyrrolopyrrole derivatives, thiadiazolopyridine derivatives, dibenzofuran derivatives, carbazole derivatives, indolocarbazole derivatives and triazine derivatives, and polymers such as polyphenylenevinylene derivatives, polyparaphenylene derivatives, polyfluorene derivatives, polyvinylcarbazole derivatives, and polythiophene derivatives. Among them, as a host to be used when the emissive layer performs triplet emission (phosphorescent emission), metal chelated oxynoid compounds, dibenzofuran derivatives, dibenzothiophene derivatives, carbazole derivatives, indolocarbazole derivatives, triazine derivatives, triphenylene derivatives, etc. are suitably used.

Examples of the dopant material contained in the light-emitting material include compounds having a fused aryl ring and their derivatives, compounds having a heteroaryl ring and their derivatives, distyrylbenzene derivatives, aminostyryl derivatives, aromatic acetylene derivatives, tetraphenylbutadiene derivatives, stilbene derivatives, aldazine derivatives, pyrromethene derivatives, diketopyrrolo[3,4-c]pyrrole derivatives, coumarin derivatives, azole derivatives and their metal complexes, aromatic amine derivatives, and compounds represented by the following general formula (2). Among them, dopants containing a diamine skeleton and dopants containing a fluoranthene skeleton can further improve the luminous efficiency, and the compounds represented by the following general formula (2) can further improve the luminous efficiency and the durable life.

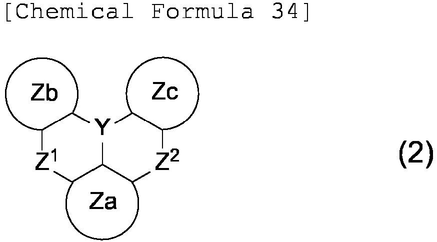

In the general formula (2), ring Za, ring Zb, and ring Zc are each independently a substituted or unsubstituted aryl ring having 6 to 30 ring-forming carbon atoms, or a substituted or unsubstituted heteroaryl ring having 5 to 30 ring-forming atoms. It is preferable that the ring Za, the ring Zb, and the ring Zc are each independently a substituted or unsubstituted aryl ring having 6 to 30 ring-forming carbon atoms. Z¹ and Z² are each independently an oxygen atom, NRa (a nitrogen atom having a substituent Ra), or a sulfur atom, provided that when Z¹ is NRa, Z¹ may or may not be bonded to ring Za or ring Zb and form a ring, and when Z² is NRa, Z² may or may not be bonded to ring Zb or ring Zc and form a ring. Ra is independently at each occurrence a substituted or unsubstituted aryl group having 6 to 30 ring-forming carbon atoms, a substituted or unsubstituted heteroaryl group having 5 to 30 ring-forming atoms, or a substituted or unsubstituted alkyl group having 1 to 30 carbon atoms. It is preferable that Z¹ and Z² are both NRa, and Ra is a substituted or unsubstituted aryl group having 6 to 30 ring-forming carbon atoms. Y is a boron atom, a phosphorus atom, SiRb (a silicon atom having substituent Rb), P=O, or P=S. Rb is independently at each occurrence a substituted or unsubstituted aryl group having 6 to 30 ring-forming carbon atoms, a substituted or unsubstituted heteroaryl group having 5 to 30 ring-forming atoms, or a substituted or unsubstituted alkyl group having 1 to 30 carbon atoms. Y is preferably a boron atom. In all the groups described above, when a group is substituted, the substituent is preferably an alkyl group, a cycloalkyl group, a heterocyclic group, an alkenyl group, a cycloalkenyl group, an alkynyl group, an aryl group, a heteroaryl group, a hydroxyl group, a thiol group, an alkoxy group, an alkylthio group, an aryl ether group, an aryl thioether group, a halogen, a cyano group, a formyl group, an acyl group, a carboxyl group, an ester group, an amide group, a sulfonyl group, a sulfonate ester group, a sulfonamide group, an amino group, a nitro group, a silyl group, a siloxanyl group, a boryl group, or an oxo group. These substituents may be further substituted with the substituents described above.

Examples of the alkyl group and the alkoxy group include those enumerated as examples of the substituent in the general formula (1).

The cycloalkyl group denotes a saturated alicyclic hydrocarbon group, such as a cyclopropyl group, a cyclohexyl group, a norbornyl group, or an adamantyl group, and the cycloalkyl group may or may not have a substituent. There is no particular limitation on the number of ring-forming carbon atoms, and the number of ring-forming carbon atoms is preferably in the range of 3 or more and 20 or less.

The heterocyclic group denotes an aliphatic ring having an atom other than carbon in the ring, such as a pyran ring, a piperidine ring, or a cyclic amide, and the heterocyclic group may or may not have a substituent. There is no particular limitation on the number of ring-forming atoms, and the number of ring-forming atoms is preferably in the range of 3 or more and 20 or less.

The alkenyl group denotes an unsaturated aliphatic hydrocarbon group including a double bond, such as a vinyl group, an allyl group, or a butadienyl group, and the alkenyl group may or may not have a substituent. The number of carbon atoms in the alkenyl group is not particularly limited, and is preferably in the range of 2 or more and 20 or less.

The cycloalkenyl group denotes an unsaturated alicyclic hydrocarbon group including a double bond, such as a cyclopentenyl group, a cyclopentadienyl group, or a cyclohexenyl group, and the cycloalkenyl group may or may not have a substituent.

The alkynyl group denotes an unsaturated aliphatic hydrocarbon group including a triple bond, such as an ethynyl group, and the alkynyl group may or may not have a substituent. The number of carbon atoms in the alkynyl group is not particularly limited, and is preferably in the range of 2 or more and 20 or less.

The aryl group denotes an aromatic hydrocarbon group, such as a phenyl group, a biphenyl group, a terphenyl group, a naphthyl group, a fluorenyl group, a benzofluorenyl group, a dibenzofluorenyl group, a phenanthryl group, an anthracenyl group, a benzophenanthryl group, a benzoanthracenyl group, a chrysenyl group, a pyrenyl group, a fluoranthenyl group, a triphenylenyl group, a benzofluoranthenyl group, a dibenzoanthracenyl group, a perylenyl group, or a helicenyl group. Among these groups, a phenyl group, a biphenyl group, a terphenyl group, a naphthyl group, a fluorenyl group, a phenanthryl group, an anthracenyl group, a pyrenyl group, a fluoranthenyl group, and a triphenylenyl group are preferable. The aryl group may or may not have a substituent. The number of carbon atoms in the aryl group is not particularly limited, and is preferably in the range of 6 or more and 40 or less, and more preferably 6 or more and 30 or less.

In a substituted phenyl group, when the phenyl group has substituents on two adjacent carbon atoms of the phenyl group, the substituents may form a ring structure together. The resulting group may correspond to any one or more of a "substituted phenyl group", an "aryl group having a structure in which two or more rings are fused", and a "heteroaryl group having a structure in which two or more rings are fused" depending on the structure.

The heteroaryl group denotes a cyclic aromatic group having one or a plurality of atoms other than carbon in the ring, such as a pyridyl group, a furanyl group, a thiophenyl group, a quinolinyl group, an isoquinolinyl group, a pyrazinyl group, a pyrimidyl group, a pyridazinyl group, a triazinyl group, a naphthyridinyl group, a cinnolinyl group, a phthalazinyl group, a quinoxalinyl group, a quinazolinyl group, a benzofuranyl group, a benzothiophenyl group, an indolyl group, a dibenzofuranyl group, a dibenzothiophenyl group, a carbazolyl group, a benzocarbazolyl group, a carbolinyl group, an indolocarbazolyl group, a benzofurocarbazolyl group, a benzothienocarbazolyl group, a dihydroindenocarbazolyl group, a benzoquinolinyl group, an acridinyl group, a dibenzoacridinyl group, a benzoimidazolyl group, an imidazopyridyl group, a benzoxazolyl group, a benzothiazolyl group, or a phenanthrolinyl group. The term "naphthyridinyl group" refers to any one of a 1,5-naphthyridinyl group, a 1,6-naphthyridinyl group, a 1,7-naphthyridinyl group, a 1,8-naphthyridinyl group, a 2,6-naphthyridinyl group, and a 2,7-naphthyridinyl group. The heteroaryl group may or may not have a substituent. The number of ring-forming atoms in the heteroaryl group is not particularly limited, and is preferably in the range of 3 or more and 40 or less, and more preferably 3 or more and 30 or less.

The term "alkylthio group" refers to a group in which the oxygen atom of the ether bond of an alkoxy group is substituted by a sulfur atom. The alkylthio group may or may not have a substituent. The number of carbon atoms in the alkylthio group is not particularly limited, and is preferably in the range of 1 or more and 20 or less.

The aryl ether group denotes a functional group having an aromatic hydrocarbon group bonded via an ether bond, such as a phenoxy group, and the aryl ether group may have a substituent or no substituent. The number of carbon atoms in the aryl ether group is not particularly limited, and is preferably in the range of 6 or more and 40 or less.

The term "aryl thioether group" refers to a functional group in which an oxygen atom in an ether linkage in an aryl ether group is replaced by a sulfur atom, and may or may not have a substituent. The number of carbon atoms in the aryl thioether group is not particularly limited, and is preferably in the range of 6 or more and 40 or less.

The halogen refers to fluorine, chlorine, bromine, or iodine.

The acyl group denotes a functional group having an alkyl group, a cycloalkyl group, an alkenyl group, an alkynyl group, an aryl group, or a heteroaryl group bonded via a carbonyl group, such as an acetyl group, a propionyl group, a benzoyl group, or an acrylyl group, and the acyl group may or may not have a substituent. The number of carbon atoms in the acyl group is not particularly limited, and is preferably 2 or more and 40 or less, and more preferably 2 or more and 30 or less.

The ester group denotes a functional group having, for example, an alkyl group, a cycloalkyl group, an aryl group, or a heteroaryl group bonded thereto via an ester linkage, and the ester group may or may not have a substituent. The number of carbon atoms in the ester group is not particularly limited, and is preferably in the range of 1 or more and 20 or less. More specific examples include a methyl ester group such as a methoxycarbonyl group, an ethyl ester group such as an ethoxycarbonyl group, a propyl ester group such as a propoxycarbonyl group, a butyl ester group such as a butoxycarbonyl group, an isopropyl ester group such as an isopropoxymethoxycarbonyl group, a hexyl ester group such as a hexyloxycarbonyl group, and a phenyl ester group such as a phenoxycarbonyl group.

The amide group denotes a functional group having, for example, an alkyl group, a cycloalkyl group, an aryl group, or a heteroaryl group bonded thereto via an amide linkage, and the amide group may or may not have a substituent. The number of carbon atoms in the amide group is not particularly limited, and is preferably in the range of 1 or more and 20 or less. More specific examples include a methylamide group, an ethylamide group, a propylamide group, a butyramide group, an isopropylamide group, a hexylamide group, and a phenylamide group.

The sulfonyl group denotes a functional group having, for example, an alkyl group, a cycloalkyl group, an aryl group, or a heteroaryl group bonded thereto via a -S(=O)₂-linkage, and the sulfonyl group may or may not have a substituent. The number of carbon atoms in the sulfonyl group is not particularly limited, and is preferably in the range of 1 or more and 20 or less.

The sulfonate ester group denotes a functional group having, for example, an alkyl group, a cycloalkyl group, an aryl group, or a heteroaryl group bonded thereto via a sulfonate ester linkage, and the sulfonate ester group may or may not have a substituent. Here, the term "sulfonate ester linkage" refers to a linkage in which a carbonyl moiety in an ester linkage, namely, -C(=O)- is replaced by a sulfonyl moiety, namely, -S(=O)₂-. The number of carbon atoms in the sulfonate ester group is not particularly limited, and is preferably in the range of 1 or more and 20 or less.

The sulfonamide group denotes a functional group having, for example, an alkyl group, a cycloalkyl group, an aryl group, or a heteroaryl group bonded thereto via a sulfonamide linkage, and the sulfonamide group may or may not have a substituent. Here, the sulfonamide linkage refers to a linkage in which a carbonyl moiety in an ester linkage, namely, -C(=O)- is replaced by a sulfonyl moiety, namely, -S(=O)₂-. The number of carbon atoms in the sulfonamide group is not particularly limited, and is preferably in the range of 1 or more and 20 or less.

The amino group may or may not have a substituent. The number of carbon atoms in the amino group is not particularly limited, and is preferably in the range of 2 or more and 50 or less, more preferably 6 or more and 40 or less, and particularly preferably 6 or more and 30 or less.

The silyl group denotes a functional group having a substituted or unsubstituted silicon atom bonded, and denotes, for example, an alkylsilyl group such as a trimethylsilyl group, a triethylsilyl group, a tertbutyldimethylsilyl group, a propyldimethylsilyl group, or a vinyldimethylsilyl group, or an arylsilyl group such as a phenyldimethylsilyl group, a tert-butyldiphenylsilyl group, a triphenylsilyl group, or a trinaphthylsilyl group. The silyl group may or may not have a substituent. The number of carbon atoms in the silyl group is not particularly limited, and is preferably in the range of 1 or more and 30 or less.

The term "siloxanyl group" refers to a silicon compound group that is bonded via an ether linkage, such as a trimethylsiloxanyl group. The siloxanyl group may or may not have a substituent.

The boryl group may or may not have a substituent.

Examples of the compound represented by the general formula (2) include the following compounds.

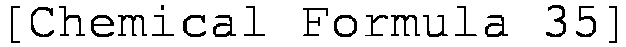

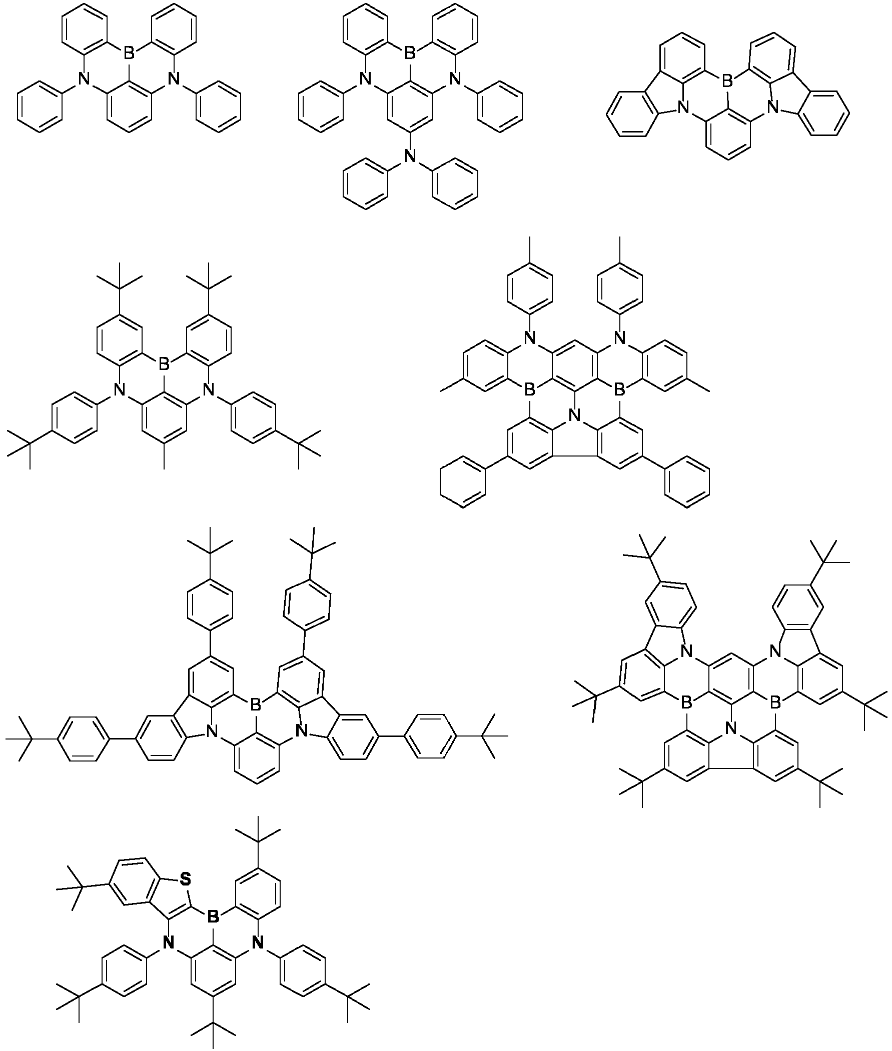

In the organic EL element according to an embodiment of the present invention, it is also preferable that the emissive layer contains a triplet light-emitting material.

The dopant to be used when the emissive layer performs triplet emission (phosphorescent emission) is preferably a metal complex compound containing at least one metal selected from the group consisting of iridium (Ir), ruthenium (Ru), palladium (Pd), platinum (Pt), osmium (Os), and rhenium (Re). The ligand preferably has a nitrogen-containing aromatic heterocyclic ring such as a phenylpyridine skeleton, a phenylquinoline skeleton, or a carbene skeleton. However, the complex is not limited thereto, and an appropriate complex is selected considering the required emission color, element performance, and relationship with the host compound. Examples of such a complex specifically include tris-(2-phenylpyridyl) iridium complex, tris-{2-(2-thiophenyl)pyridyl} iridium complex, tris-{2-(2-benzothiophenyl)pyridyl} iridium complex, tris-(2-phenylbenzothiazole) iridium complex, tris-(2-phenylbenzooxazole) iridium complex, tris-benzoquinoline iridium complex, bis(2-phenylpyridyl) (acetylacetonato) iridium complex, bis{2-(2-thiophenyl)pyridyl} iridium complex, bis{2-(2-benzothiophenyl)pyridyl} (acetylacetonato) iridium complex, bis(2-phenylbenzothiazole) (acetylacetonato) iridium complex, bis(2-phenylbenzooxazole) (acetylacetonato) iridium complex, bisbenzoquinoline (acetylacetonato) iridium complex, bis{2-(2,4-difluorophenyl)pyridyl} (acetylacetonato) iridium complex, tetraethylporphyrin platinum complex, {tris-(thenoyltrifluoroacetone) mono(1,10-phenanthroline)} europium complex, {tris-(thenoyltrifluoroacetone) mono(4,7-diphenyl-1,10-phenanthroline)} europium complex, {tris-(1,3-diphenyl-1,3-propanedione) mono(1,10-phenanthroline)} europium complex, and tris-acetylacetone terbium complex. The phosphorescent dopants described in JP 2009-130141 A are also suitably used. An iridium complex or a platinum complex is preferable, and the luminous efficiency can thereby be further improved.

As to the triplet light-emitting material to be used as the dopant material, the emissive layer may contain only one triplet light-emitting material, or two or more types thereof may be used in combination. When two or more triplet light-emitting materials are used, the total weight of the dopant material is preferably 30% by weight or less, more preferably 20% by weight or less with respect to the host material.

The host and the dopant preferable in a triplet emission system are not particularly limited, and specific examples thereof include the following.

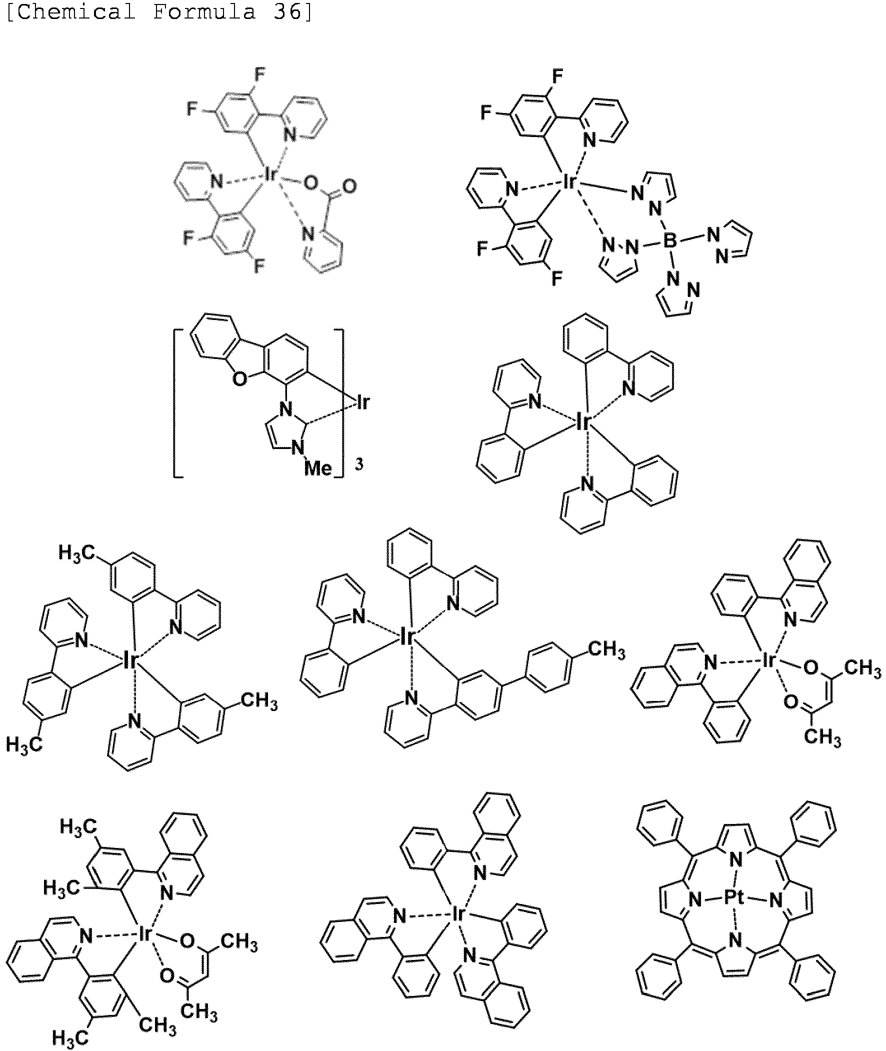

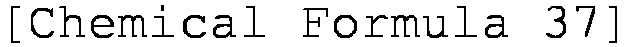

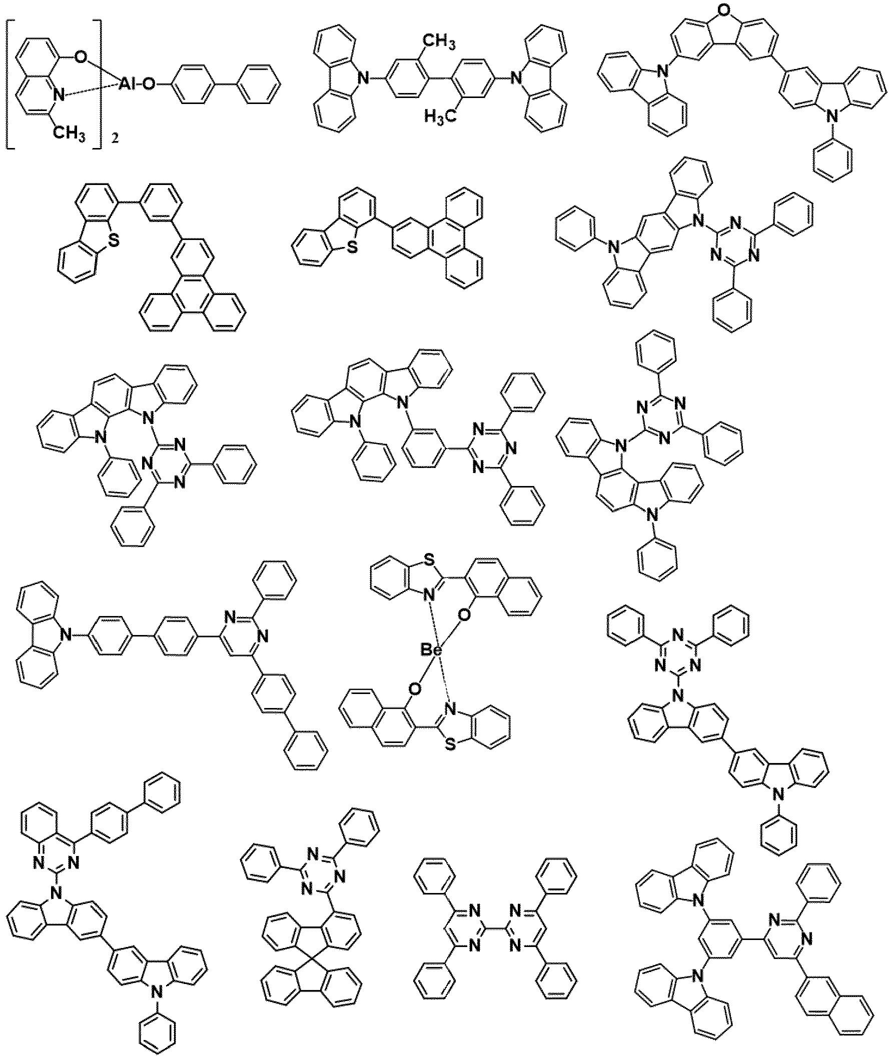

It is also preferred that the emissive layer contains a thermally activated delayed fluorescence material. Thermally activated delayed fluorescence is explained in "State-of-the-Art Organic Light-Emitting Diodes" (edited by Chihaya Adachi and Hiroshi Fujimoto, published by CMC Publishing Co., Ltd.), pages 87-103. The document explains that when energy levels in an excited singlet state and an excited triplet state of a fluorescent light-emitting material are brought close to each other, reverse energy transfer to the excited singlet state from the excited triplet state normally with a low transition probability occurs with high efficiency, so that thermally activated delayed fluorescence (TADF) is generated. Further, the mechanism of generation of delayed fluorescence is illustrated in Fig. 5 in the document. The light emission of delayed fluorescence can be determined by transient PL (photo luminescence) measurement.

Thermally activated delayed fluorescence materials are also commonly referred to as TADF materials. In the thermally activated delayed fluorescence material, the thermally activated delayed fluorescence may be exhibited with a single material or a plurality of materials. When the material is composed of a plurality of materials, the material may be used in the form of a mixture, or layers composed of the respective materials may be laminated and used. As the thermally activated delayed fluorescence material, publicly known materials can be used. Specific examples of the material include, but are not limited to, benzonitrile derivatives, triazine derivatives, disulfoxide derivatives, carbazole derivatives, indolocarbazole derivatives, dihydrophenazine derivatives, thiazole derivatives, and oxadiazole derivatives.

In an element in which a TADF material is contained in an emissive layer, the emissive layer preferably further contains a fluorescent dopant. This is because triplet excitons are converted into singlet excitons by the TADF material, and the fluorescent dopant receives the singlet excitons, whereby higher luminous efficiency and longer durable life can be achieved.

### (Electron Transporting Layer)

In the present invention, the electron transporting layer is a layer into which electrons are injected from the cathode and which transports the electrons. The electron transporting layer is desired to have a high electron injection efficiency and efficiently transport injected electrons. Therefore, it is preferable that the material forming the electron transporting layer be a substance that has high electron affinity and high electron mobility, is superior in stability, and hardly generates impurities that serve as a trap, at the time of production and use. In particular, in the case of laminating layers in a large film thickness, the film quality easily degrades due to crystallization of a compound having a low molecular weight or the like, and therefore, to maintain stable film quality, a compound having a molecular weight of 400 or more is preferred. In consideration of the balance of transportation between holes and electrons, however, if the electron transporting layer mainly plays the role of efficiently preventing holes from the anode from flowing to the cathode without recombination, the effect of improving the luminous efficiency is equivalent to the case where the electron transporting layer is made from a material having high electron transport ability even if the electron transporting layer is made from a material whose electron transporting ability is not so high. Therefore, the electron transporting layer in the present invention includes, as a synonym, a hole inhibition layer capable of efficiently inhibiting the transfer of holes, and the hole inhibition layer and the electron transporting layer each may be a single layer or may be formed of a plurality of materials laminated.

As the electron transporting material to be used for the electron transporting layer, a publicly known material can be used. Examples thereof include fused polycyclic aromatic derivatives, styryl-based aromatic ring derivatives, quinone derivatives, phosphorus oxide derivatives, and various metal complexes such as quinolinol complexes, benzoquinolinol complexes, hydroxyazole complexes, azomethine complexes, tropolone metal complexes, and flavonol metal complexes. It is preferable to use a compound that includes an element selected from among carbon, hydrogen, nitrogen, oxygen, silicon, and phosphorus and has a heteroaryl ring structure including electron-accepting nitrogen because the driving voltage is further reduced and further efficient light emission can be obtained with such a compound.

Here, the electron-accepting nitrogen denotes a nitrogen atom forming a multiple bond with an adjacent atom. The multiple bond has an electron-accepting property because a nitrogen atom has a high electron negativity. Therefore, an aromatic heterocyclic ring including electron-accepting nitrogen has a high electron affinity. An electron transporting material having electron-accepting nitrogen facilitates reception of electrons from a cathode having a high electron affinity and makes driving at a lower voltage possible. Furthermore, the supply of electrons to the emissive layer increases to enhance the recombination probability, and as a result, the luminous efficiency is further improved.

Examples of the heteroaryl ring including electron-accepting nitrogen include a triazine ring, a pyridine ring, a pyrazine ring, a pyrimidine ring, a quinoline ring, a quinoxaline ring, a quinazoline ring, a naphthyridine ring, a pyrimidopyrimidine ring, a benzoquinoline ring, a phenanthroline ring, an imidazole ring, an oxazole ring, an oxadiazole ring, a triazole ring, a thiazole ring, a thiadiazole ring, a benzoxazole ring, a benzothiazole ring, a benzimidazole ring, and a phenanthroimidazole ring.

Examples of the compound having such a heteroaryl ring structure include pyridine derivatives, triazine derivatives, quinazoline derivatives, pyrimidine derivatives, benzimidazole derivatives, benzoxazole derivatives, benzthiazole derivatives, oxadiazole derivatives, thiadiazole derivatives, triazole derivatives, pyrazine derivatives, phenanthroline derivatives, quinoxaline derivatives, quinoline derivatives, benzoquinoline derivatives, oligopyridine derivatives, quinoxaline derivatives, and naphthyridine derivatives. Among them, imidazole derivatives, oxadiazole derivatives, triazole derivatives, phenanthroline derivatives, benzoquinoline derivatives, bipyridine derivatives, terpyridine derivatives, and naphthyridine derivatives are preferably used from the viewpoint of electron transporting ability.

In addition, the fact that these derivatives have a fused polycyclic aromatic skeleton is more preferable because the glass transition temperature is increased and the electron mobility is also increased, so that the driving voltage of an organic EL element can be further reduced. Furthermore, in consideration of further improvement in the durable life of an element, ease of synthesis, and availability of raw materials, it is more preferable that the fused polycyclic aromatic skeleton be a fluoranthene skeleton, an anthracene skeleton, a pyrene skeleton, or a phenanthroline skeleton.

Preferable electron transporting materials are not particularly limited, and examples thereof specifically include the following.

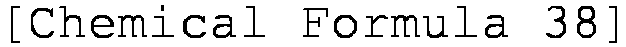

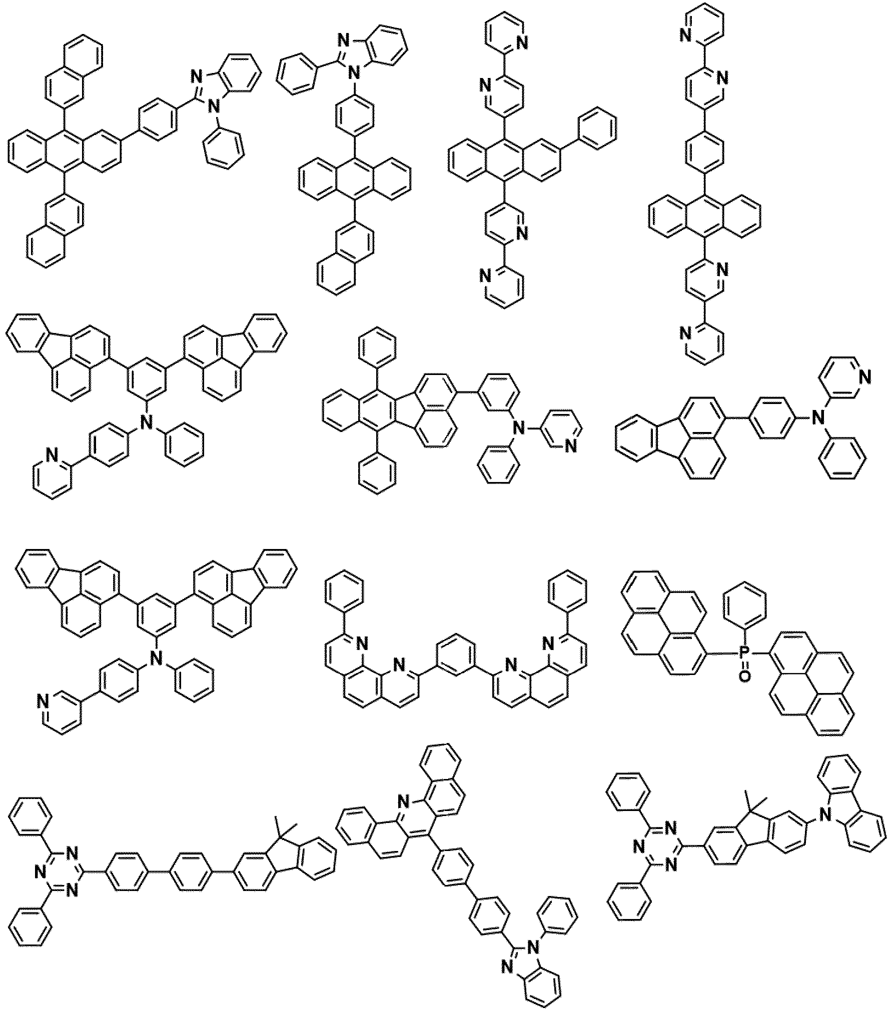

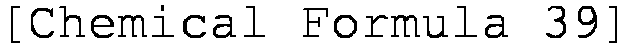

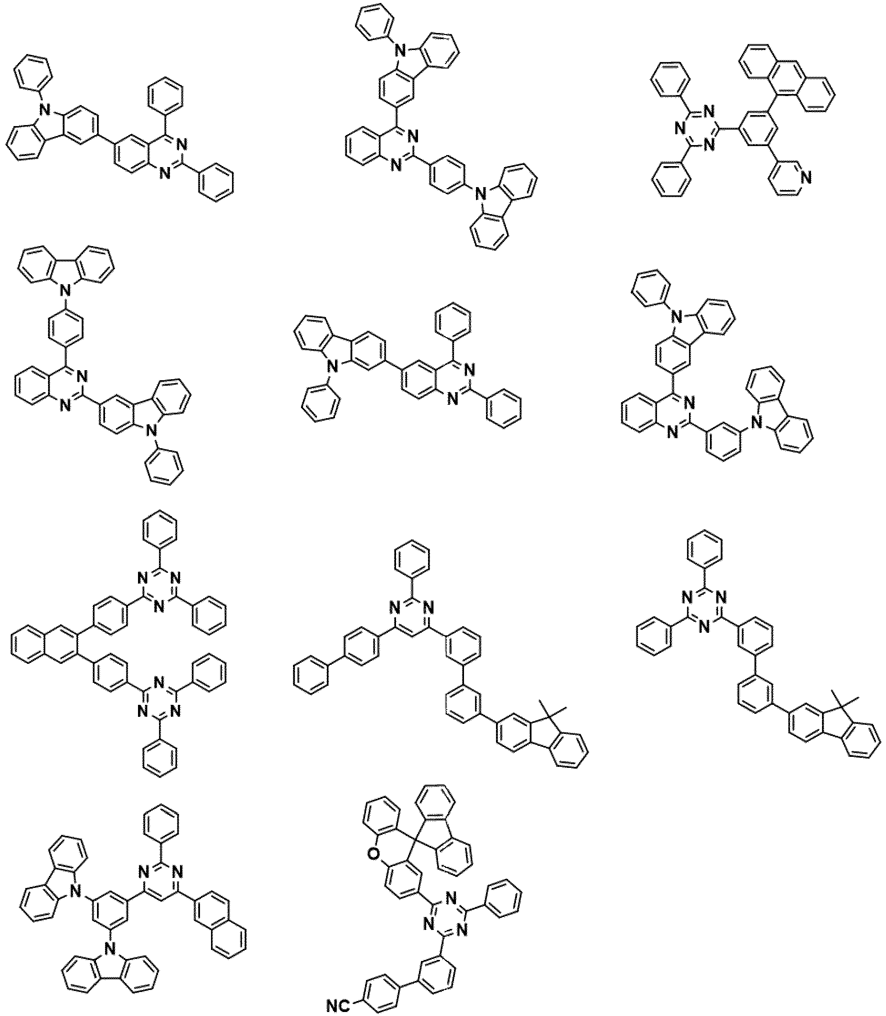

In addition, the compound represented by the general formula (1) is also preferable because it has a high electron transporting property and exhibits superior properties as an electron transporting layer.

As to the electron transporting material described above, a single material thereof may be used alone, two or more electron transporting materials described above may be used in combination, or one or more other electron transporting materials may be used in combination with the electron transporting material described above. The electron transporting material may contain a donor compound. Here, the donor compound denotes a compound that facilitates electron injection from the cathode or the electron injection layer into the electron transporting layer through improvement in the electron injection barrier, and improves the electroconductivity of the electron transporting layer.

From the viewpoint of improvement in electron transporting characteristics with a low work function, the electron transporting material preferably contains an alkali metal atom, an alkaline earth metal atom, or a rare earth metal atom as the donor compound. In particular, in terms of the fact that the driving voltage of an organic EL can be further reduced, it is more preferable that the electron transporting layer contains an alkali metal atom or a rare earth metal atom as the donor compound.

In addition, the donor compound is preferably an inorganic salt or in a state of a complex of a metal with an organic substance rather than a simple metal because such a material is easily deposited in vacuum and is superior in handleability. Furthermore, it is more preferable that the donor compound is in the state of a complex of a metal with an organic substance in terms of facilitating handling in the atmosphere and facilitating adjustment of the addition concentration. Examples of the inorganic salt include oxides, nitrides, fluorides, and carbonates. Regarding the complex with an organic substance, preferable examples of the organic substance include quinolinol, benzoquinolinol, pyridylphenol, flavonol, hydroxyimidazopyridine, hydroxybenzazole, and hydroxytriazole. Among them, in terms of the fact that the driving voltage of the organic EL element can be further reduced, the electron transporting layer preferably contains a complex of an alkali metal with an organic substance as the donor compound. Furthermore, in terms of ease of synthesis and thermal stability, a complex of lithium with an organic substance is more preferable as the donor compound, and lithium quinolinol (Liq), which is available at a relatively low cost, is particularly preferable.

The ionization potential of the electron transporting layer is not particularly limited, but is preferably 5.6 eV or more and 8.0 eV or less, and more preferably 5.6 eV or more and 7.0 eV or less.

The method for forming each of the aforementioned layers constituting the organic EL element is not particularly limited and may be resistance heating deposition, electron beam deposition, sputtering, a molecular lamination method, a coating method, or the like, but usually, resistance heating deposition or electron beam deposition is preferred from the viewpoint of element properties.

### (Electron Injection Layer)

In the present invention, an electron injection layer may be provided between the cathode and the electron transporting layer. The electron injection layer is interposed generally for the purpose of assisting injection of electrons from the cathode to the electron transporting layer. When the electron injection layer is interposed, a compound having a heteroaryl ring structure including electron-accepting nitrogen may be used, or a layer containing the above-described donor material may be used.

An inorganic substance such as an insulator or a semiconductor can also be used for the electron injection layer, and publicly known materials can be used. The use of such a material can inhibit a short-circuit of the organic EL element, and can improve electron injection property.

As such an insulator, at least one metal compound selected from the group consisting of alkali metal chalcogenides, alkaline earth metal chalcogenides, halides of alkali metal, and halides of alkaline earth metal is preferable.

A complex of an organic substance with a metal is also suitably used. When a complex of an organic substance with a metal is used in the electron injection layer, the film thickness can be easily adjusted. Preferable examples of the organic substance in the organic metal complex include quinolinol, benzoquinolinol, pyridylphenol, flavonol, hydroxyimidazopyridine, hydroxybenzazole, and hydroxytriazole.

In addition, the layer containing the compound represented by the general formula (1) also has a high electron injection property, and is also preferable because it exhibits superior properties as an electron injection layer. Furthermore, the compound represented by the general formula (1) is preferably doped with the alkali metal or rare earth metal, and such doping can further reduce the driving voltage and further improve the durable life. That is, the electron injection layer preferably further contains an alkali metal atom or a rare earth metal atom.

### (Charge Generation Layer)

The charge generation layer in the present invention is generally formed of a double layer, and can be used specifically in the form of a p-n junction charge generation layer composed of an n-type charge generation layer and a p-type charge generation layer. The p-n junction charge generation layer generates a charge or separates a charge into a hole and an electron when a voltage is applied in the organic EL element, and the hole and the electron are injected into the emissive layer via the hole transporting layer and the electron transporting layer. Specifically, in the organic EL element in which the emissive layers are laminated, the p-n junction charge generation layer functions as a charge generation layer that is an intermediate layer. The n-type charge generation layer supplies electrons to the first emissive layer present on the anode side, and the p-type charge generation layer supplies holes to the second emissive layer present on the cathode side. Therefore, in the organic EL element in which a plurality of emissive layers are laminated, the luminous efficiency can be further improved, the driving voltage can be reduced, and the durable life of the element can also be improved.

The n-type charge generation layer includes an n-type dopant and a host, and as the n-type dopant and the host, conventional materials can be used. For example, an alkali metal, an alkaline earth metal, or a rare earth metal can be used as the n-type dopant. In addition, as the host, a compound having a nitrogen-containing aromatic heterocyclic ring such as a phenanthroline derivative or an oligopyridine derivative can be used. In particular, a compound represented by the general formula (1) and phenanthroline dimer are preferable because they exhibit superior properties as a host of the n-type charge generation layer.

As one aspect of the charge generation layer, it is preferable to further contain phenanthroline dimer in addition to the compound represented by the general formula (1). Examples of the phenanthroline dimer include the following compounds.

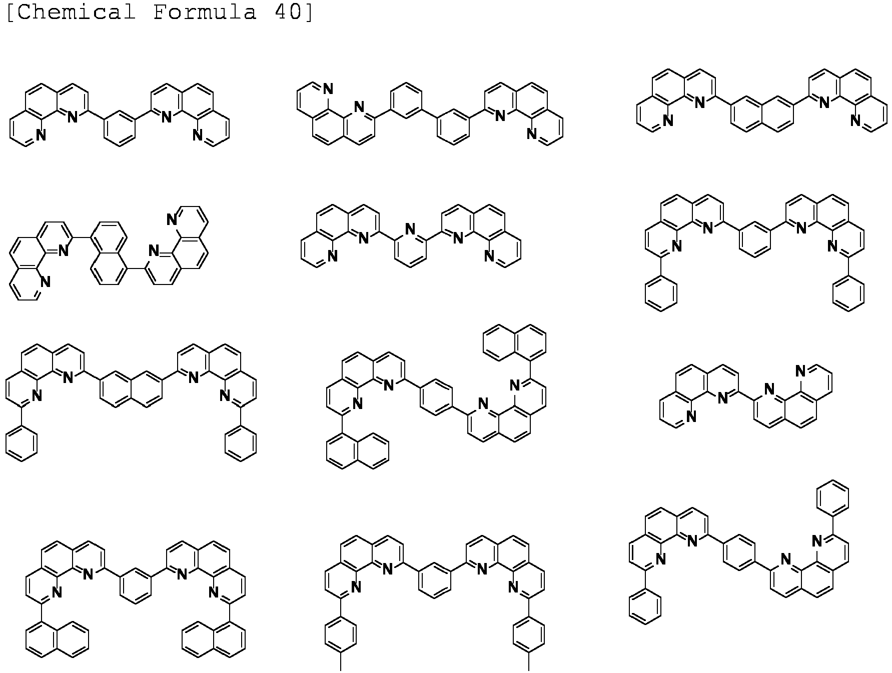

As one aspect of the charge generation layer, it is preferable to further contain an alkali metal atom or a rare earth metal atom in addition to the compound represented by the general formula (1). Li is particularly preferable as the alkali metal atom. Yb is particularly preferable as the rare earth metal atom.

As one aspect of the charge generation layer, a configuration further containing phenanthroline dimer and an alkali metal or rare earth metal atom in addition to the compound represented by the general formula (1) is also preferable.

The p-type charge generation layer includes a p-type dopant and a host, and as the p-type dopant and the host, conventional materials can be used. For example, tetrafluorene-7,7,8,8-tetracyanoquinodimethane (F4-TCNQ), a tetracyanoquinodimethane derivative, a radialene derivative, iodine, FeCl₃, FeF₃, or SbCl₅ can be used as the p-type dopant. The p-type dopant is preferably a radialene derivative. The host is preferably an arylamine derivative.

The thickness of the organic layer depends on the resistance value of the emissive substance and is not limited, but is preferably 1 to 1,000 nm. The emissive layer, the electron transporting layer, and the hole transporting layer each preferably have a thickness of 1 nm or more and 200 nm or less, and more preferably 5 nm or more and 100 nm or less.

The organic EL element according to an embodiment of the present invention has a function of converting electrical energy into light. Here, as the electrical energy, a direct current is mainly used, but a pulse current or an alternating current can also be used. A current value and a voltage value are not particularly limited, but when the power consumed and life of the element are considered, they should be selected such that the maximum luminance is obtained by energy as low as possible.

The light-emitting element according to an embodiment of the present invention is suitably used, for example, as a display device such as a display that displays in a matrix and/or segment system. That is, a light-emitting element containing the compound according to an embodiment of the present invention is suitably used as a display device.

The light-emitting element according to an embodiment of the present invention is also preferably used as a backlight for various devices and the like. The backlight is mainly used for the purpose of improving the visibility of a display device such as a display that does not emit light, and is used in liquid crystal displays, watches, audio devices, automobile panels, display boards, signs, and the like. In particular, the light-emitting element of the present invention is preferably used in a backlight for a liquid crystal display, particularly for a personal computer that is studied for reduction in the thickness, and a backlight thinner and lighter than conventional ones can be provided.

The light-emitting element according to an embodiment of the present invention is also preferably used as various illumination devices. That is, the light-emitting element containing the compound according to an embodiment of the present invention is suitably used as an illumination device. The light-emitting element according to an embodiment of the present invention can achieve both high luminous efficiency and high color purity, and further can achieve reduction in the thickness and the weight, so that an illumination device having a low power consumption, a bright emitted color, and a high design property in combination can be realized therewith.

### EXAMPLES

Hereinafter, the present invention will be described by way of Examples, but the present invention is not limited thereto.

### Synthesis Example 1: Synthesis of Compound 2

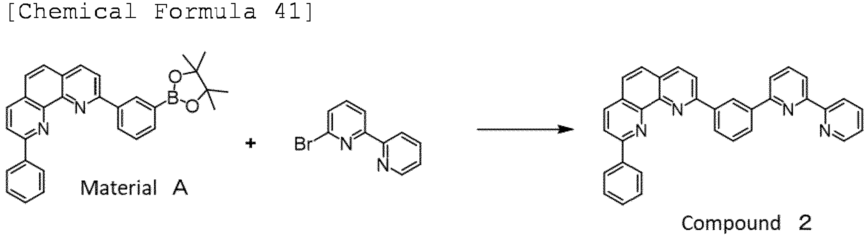

A mixed solution of 10 g of raw material A, 4.9 g of 6-bromo-2,2'-bromobipyridine, 150 mg of dichlorobis(triphenylphosphine palladium) dichloride, 40 ml of a 0.75 M aqueous potassium carbonate solution, and 210 ml of dimethoxyethane was heated and stirred under reflux for 5 hours under a nitrogen flow. After cooling to room temperature, water was added, filtration was performed, and the residue was washed with methanol and vacuum dried. The resulting solid was subjected to removal of a catalyst using activated carbon, and the solvent was removed by evaporation. The resulting solid was recrystallized from toluene, and then vacuum-dried, affording 7.2 g of Compound 2.

The resulting Compound 7 was subjected to sublimation purification at about 290°C under a pressure of 1 × 10⁻³ Pa using an oil diffusion pump. The HPLC purity (area% at a measurement wavelength of 254 nm) of Compound 2 before the sublimation purification and that after the sublimation purification were both 99.9%.

After the sublimation purification, the structure of Compound 2 was identified by mass spectrum (MS) analysis and ¹H-NMR analysis. The analysis results are shown below.
MS (m/z): 487 [M+H]⁺
¹H-NMR (400 MHz, CDCl₃)δ: 9.37 (m, 1H), 8.72-8.74 (m, 2H), 8.51-8.54 (m, 3H), 8.45-8.47 (m, 1H), 8.29-8.41 (m, 3H), 8.17-8.20 (m, 1H), 8.09-8.12 (m, 1H), 7.98-8.02 (m, 1H), 7.82-7.86 (m, 3H), 7.74-7.76 (m, 1H), 7.46-7.55 (m, 3H), 7.35-7.30 (m, 1H), 7.26 (m, 1H).

### Synthesis Example 2: Synthesis of Compound 6

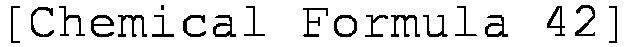

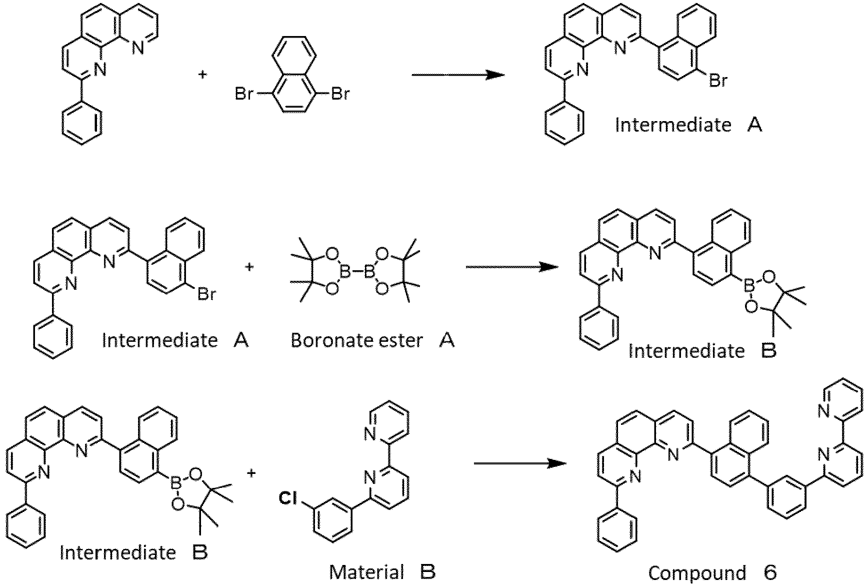

75 ml of n-butyllithium (1.6 M solution in hexane) was added dropwise to a mixed solution of 34.3 g of 1,4-dibromonaphthalene and 130 ml of tetrahydrofuran at 0°C under a nitrogen flow. The mixture was stirred at 0°C for 30 minutes, and then added dropwise at 0°C to a mixed liquid of 25.5 g of 2-phenyl-1,10-phenanthroline and 150 ml of THF. After the temperature was raised to room temperature, 150 ml of a saturated aqueous ammonium chloride solution was added to the reaction solution. The organic layer was extracted with ethyl acetate and evaporated. To the resulting solid were added 340 ml of dichloromethane and 15.6 g of manganese dioxide, and the mixture was stirred at room temperature for 1 hour. The reaction solution was filtered, and the filtrate was concentrated by evaporation, then the residual solid was washed with methanol and then vacuum-dried, affording 30.0 g of Intermediate A.

Next, a mixed solution of 15.5 g of Intermediate A, 17.0 g of boronate ester A, 10.0 g of potassium acetate, 550 mg of bis(diphenylphosphino)ferrocenedichloropalladium, and 335 ml of dimethylformamide was heated and stirred at 100°C for 1.5 hours under a nitrogen flow. After cooling to room temperature, water was added, filtration was performed, and the residue was washed with methanol and vacuum dried, affording 15.0 g of Intermediate B.

Next, a mixed solution of 15.0 g of Intermediate B, 7.9 g of raw material B, 420 mg of dichlorobis(triphenylphosphine palladium) dichloride, 50 ml of a 0.9 M aqueous potassium carbonate solution, and 300 ml of 1,4-dioxane was heated and stirred under reflux for 8 hours under a nitrogen flow. After cooling to room temperature, water was added, filtration was performed, and the residue was washed with methanol and vacuum dried. The resulting solid was subjected to removal of a catalyst using activated carbon, and the solvent was removed by evaporation. The resulting solid was recrystallized from toluene methanol, and then vacuum-dried, affording 7.5 g of Compound 6.

The resulting Compound 6 was subjected to sublimation purification at about 340°C under a pressure of 1 × 10⁻³ Pa using an oil diffusion pump. The HPLC purity (area% at a measurement wavelength of 254 nm) of Compound 6 before the sublimation purification and that after the sublimation purification were both 99.9%.

After the sublimation purification, the structure of Compound 6 was identified by mass spectrum (MS) analysis and ¹H-NMR analysis. The analysis results are shown below.
MS (m/z): 613 [M+H]⁺
¹H-NMR (400 MHz, DMSO-d₆)δ: 8.94-9.00 (m, 1H), 8.70-8.74 (m, 2H), 8.65-8.67 (m, 1H), 8.55-8.57 (m, 1H), 8.43-8.47 (m, 4H), 8.38-8.42 (m, 2H), 8.18-8.24 (m, 2H), 8.05-8.14 (m, 5H), 7.95-7.99 (m, 1H), 7.77-7.81 (m, 2H), 7.70-7.73 (m, 1H), 7.66-7.69 (m, 2H), 7.53-7.58 (m, 2H), 7.47-7.53 (m, 2H).

### Synthesis Example 3: Synthesis of Compound 10

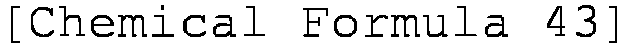

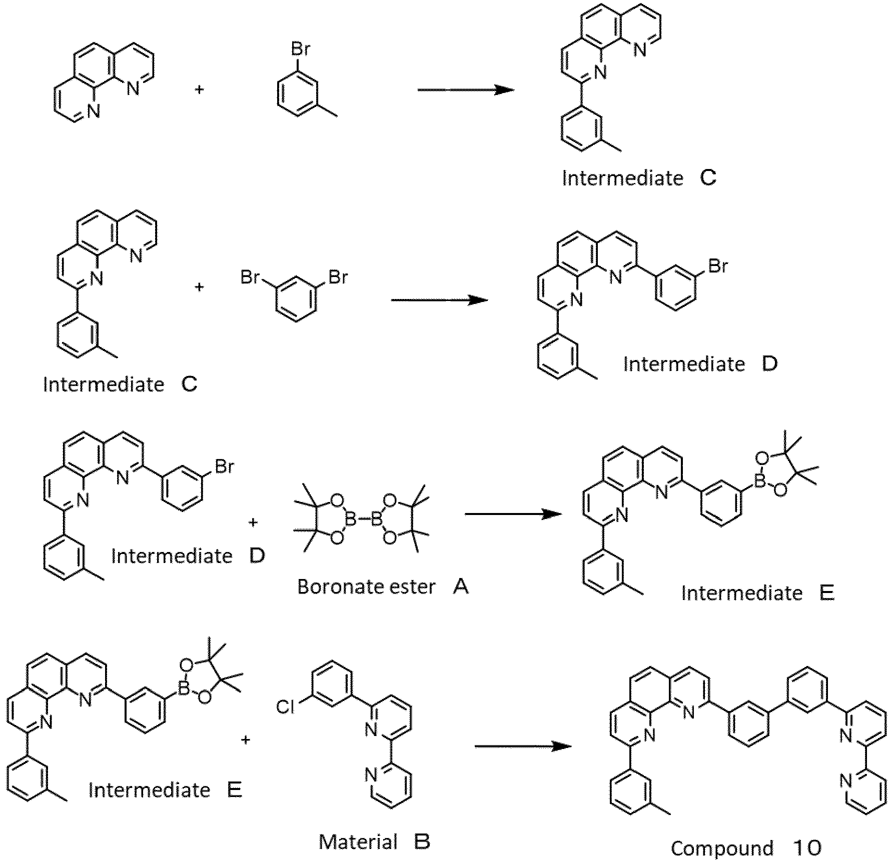

150 ml of n-butyllithium (1.6 M solution in hexane) was added dropwise to a mixed solution of 41.0 g of 3-bromotoluene and 150 ml of tetrahydrofuran at 0°C under a nitrogen flow. The mixture was stirred at 0°C for 30 minutes, and then added dropwise at 0°C to a mixed liquid of 36.0 g of 1,10-phenanthroline and 150 ml of THF. After the temperature was raised to room temperature, 250 ml of a saturated aqueous ammonium chloride solution was added to the reaction solution. The organic layer was extracted with ethyl acetate and evaporated. To the resulting solid were added 340 ml of dichloromethane and 34.7 g of manganese dioxide, and the mixture was stirred at room temperature for 1 hour. The reaction solution was filtered, and the filtrate was concentrated by evaporation, then the residual solid was washed with methanol and then vacuum-dried, affording 34.5 g of Intermediate C.

Next, 96 ml of n-butyllithium (1.6 M solution in hexane) was added dropwise to a mixed solution of 36.1 g of 1,3-dibromobenzene and 96 ml of tetrahydrofuran at 0°C under a nitrogen flow. The mixture was stirred at 0°C for 30 minutes, and then added dropwise at 0°C to a mixed liquid of 34.5 g of Intermediate C and 96 ml of THF. After the temperature was raised to room temperature, 150 ml of a saturated aqueous ammonium chloride solution was added to the reaction solution. The organic layer was extracted with ethyl acetate and evaporated. To the resulting solid were added 200 ml of dichloromethane and 22.1 g of manganese dioxide, and the mixture was stirred at room temperature for 1 hour. The reaction solution was filtered, and the filtrate was concentrated by evaporation, then the residual solid was washed with methanol and then vacuum-dried, affording 33.2 g of Intermediate D.

Next, a mixed solution of 33.0 g of Intermediate D, 27.6 g of boronate ester A, 22.8 g of potassium acetate, 570 mg of bis(diphenylphosphino)ferrocenedichloropalladium, and 780 ml of dimethylformamide was heated and stirred at 100°C for 3 hours under a nitrogen flow. After cooling to room temperature, water was added, filtration was performed, and the residue was washed with methanol and vacuum dried, affording 35.2 g of Intermediate E.

Next, a mixed solution of 30.0 g of Intermediate E, 16.9 g of raw material B, 890 mg of dichlorobis(triphenylphosphine palladium) dichloride, 140 ml of a 0.9 M aqueous potassium carbonate solution, and 630 ml of 1,4-dioxane was heated and stirred under reflux for 12 hours under a nitrogen flow. After cooling to room temperature, water was added, filtration was performed, and the residue was washed with methanol and vacuum dried. The resulting solid was subjected to removal of a catalyst using activated carbon, and the solvent was removed by evaporation. The resulting solid was recrystallized from toluene methanol, and then vacuum-dried, affording 25.5 g of Compound 10.

The resulting Compound 10 was subjected to sublimation purification at about 340°C under a pressure of 1 × 10⁻³ Pa using an oil diffusion pump. The HPLC purity (area% at a measurement wavelength of 254 nm) of Compound 10 before the sublimation purification and that after the sublimation purification were both 99.9%.

After the sublimation purification, the structure of Compound 10 was identified by mass spectrum (MS) analysis and ¹H-NMR analysis. The analysis results are shown below.
MS (m/z): 577 [M+H]⁺
¹H-NMR (400 MHz, DMSO-d₆)δ: 9.20-9.22 (m, 1H), 8.58-8.70 (m, 4H), 8.49-8.56 (m, 4H), 8.36-8.48 (m, 4H), 8.05-8.14 (m, 2H), 7.94-8.03 (m, 2H), 7.78-7.82 (m, 1H), 7.55-7.60 (m, 2H), 7.38-7.48 (m, 3H), 6.97-7.09 (m, 2H), 2.14 (s, 3H).

Next, the evaluation method in each example will be described.

### (Driving Voltage)

The elements obtained in Examples 1 to 27 and Comparative Examples 1 to 16 were each direct-current driven at 10 mA/cm², and an initial driving voltage was measured. Further, a voltage when each of the elements was direct-current driven at a current density of 10 mA/cm² for 100 hours in an environment with a temperature of 70°C was measured, and the voltage build-up amount from the initial driving voltage was calculated.

In addition, each of the organic EL elements obtained in Examples 28 to 81 and Comparative Examples 17 to 48 was turned on at a luminance of 1000 cd/m², and the initial driving voltage was measured. Further, a voltage when each of the elements was constant-current driven at a current density of 10 mA/cm² for 100 hours at room temperature was measured, and the voltage build-up amount from the initial driving voltage was calculated.

The lower the initial driving voltage is, the lower the voltage at which driving can be performed and, therefore, the better the luminous efficiency (luminance/electric power) can be evaluated to be. In addition, the smaller the voltage build-up amount is, the better the durable life can be evaluated to be.

### (External Quantum Efficiency)

Each of the organic EL elements obtained in Examples 28 to 81 and Comparative Examples 17 to 48 was turned on at a current density of 10 mA/cm², the initial driving voltage was measured, and the luminous efficiency was evaluated. The higher the external quantum efficiency is, the better the luminous efficiency can be evaluated to be.

### (Durability)

The organic EL elements obtained in Examples 28 to 81 and Comparative Examples 17 to 48 were continuously driven at a constant current of 10 mA/cm², and the time during which the luminance decreased by 20% from the initial luminance was measured and defined as durability.

### Example 1

A glass substrate on which an ITO transparent electroconductive film was deposited as an anode in a thickness of 125 nm (manufactured by GEOMATEC Co., Ltd., 11 Ω/□, sputtered product) was cut into 38 mm × 46 mm, and etched. The resulting substrate was ultrasonically washed for 15 minutes using "Semico Clean" (registered trademark) 56 (product name, manufactured by Furuuchi Chemical Corporation), and was then washed with ultra-pure water. This substrate was treated with UV-ozone for 1 hour immediately before preparation of an element, and placed in a vacuum deposition apparatus, and the apparatus was evacuated until the degree of vacuum in the apparatus was 5 × 10⁻⁴ Pa or less. Compound 1 and a metal element Yb as a dopant were vapor-deposited in a thickness of 100 nm by a resistance heating method such that the vapor deposition rate ratio was Compound 1 : Yb = 9 : 1, and thus a layer having a weight ratio of 9 : 1 was formed. Thereafter, aluminum was vapor-deposited in a thickness of 60 nm to form a cathode, and a 5 mm × 5 mm square element was produced. The thickness referred to herein is a crystal oscillation type film thickness monitor display value, and is common to other examples and comparative examples.

As a result of evaluating this element by the above-described methods, the initial driving voltage was 0.067 V, and the voltage build-up amount was 0.035 V when the element was driven at 70°C for 100 hours.

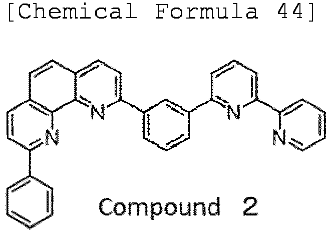

### Examples 2 to 27, Comparative Examples 1 to 16

Elements were produced in the same manner as in Example 1 except that the compound to be used, the metal element, and the vapor deposition rate ratio of the compound to the metal element were varied as shown in Table 1. The results of the example and the comparative example are shown in Table 1. Compounds 2 to 19 are the compounds shown below.

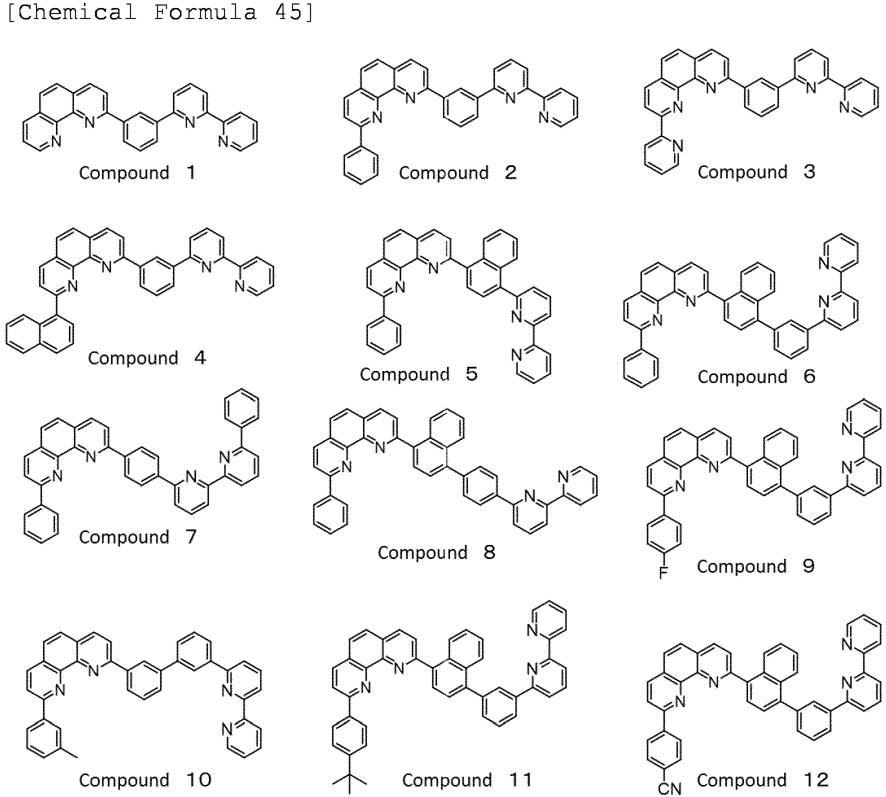

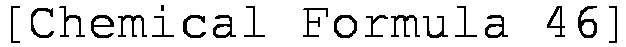

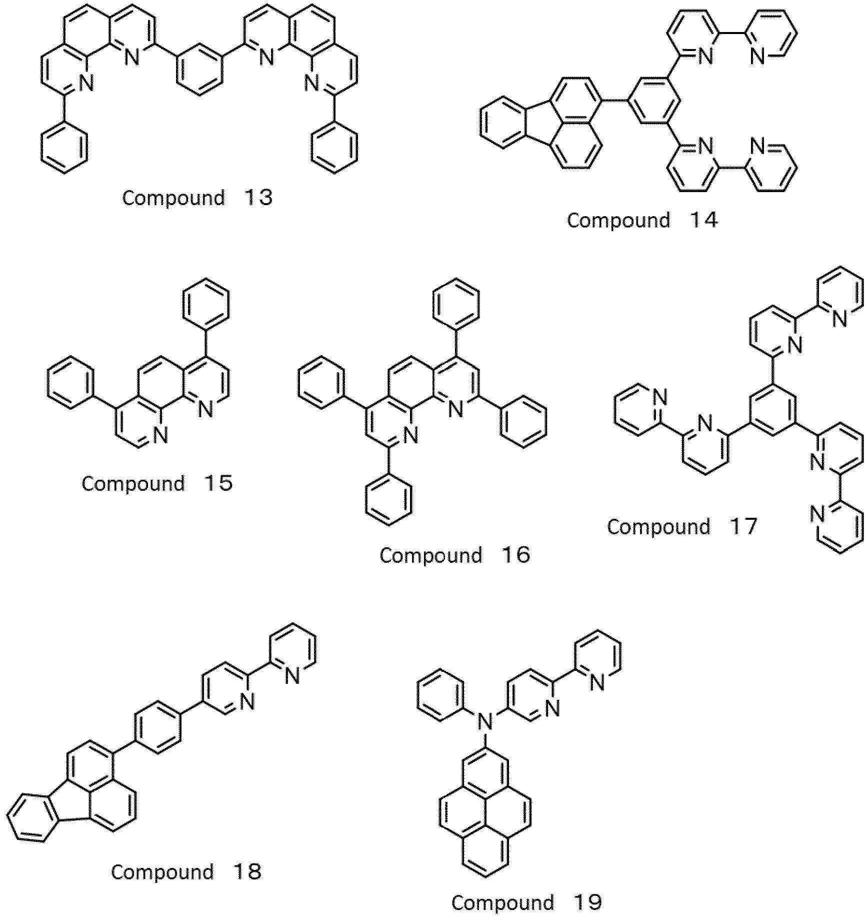

**[Table 1]**

| | Compound | Metal element (Dopant) | Vapor deposition rate ratio, compound : metal element | Voltage (V) at 10 mA/cm² | Voltage build-up (V) at 70°C, 10 mA/cm², 100 hours |
|---|---|---|---|---|---|
| Example 1 | Compound 1 | Yb | 9:1 | 0.067 | 0.035 |
| Example 2 | Compound 2 | Yb | 9:1 | 0.060 | 0.018 |
| Example 3 | Compound 3 | Yb | 9:1 | 0.073 | 0.026 |
| Example 4 | Compound 4 | Yb | 9:1 | 0.080 | 0.030 |
| Example 5 | Compound 5 | Yb | 9:1 | 0.065 | 0.015 |
| Example 6 | Compound 6 | Yb | 9:1 | 0.062 | 0.013 |
| Example 7 | Compound 7 | Yb | 9:1 | 0.061 | 0.011 |
| Example 8 | Compound 8 | Yb | 9:1 | 0.057 | 0.015 |
| Example 9 | Compound 9 | Yb | 9:1 | 0.066 | 0.012 |
| Example 10 | Compound 10 | Yb | 9:1 | 0.072 | 0.024 |
| Example 11 | Compound 11 | Yb | 9:1 | 0.068 | 0.009 |
| Example 12 | Compound 12 | Yb | 9:1 | 0.071 | 0.022 |
| Example 13 | Compound 1 | Yb | 99:1 | 0.075 | 0.038 |
| Example 14 | Compound 1 | Yb | 19:1 | 0.073 | 0.031 |
| Example 15 | Compound 1 | Yb | 7:3 | 0.070 | 0.029 |
| Example 16 | Compound 1 | Li | 99:1 | 0.082 | 0.058 |
| Example 17 | Compound 2 | Li | 99:1 | 0.071 | 0.041 |
| Example 18 | Compound 3 | Li | 99:1 | 0.077 | 0.060 |
| Example 19 | Compound 4 | Li | 99:1 | 0.089 | 0.053 |
| Example 20 | Compound 5 | Li | 99:1 | 0.075 | 0.039 |
| Example 21 | Compound 6 | Li | 99:1 | 0.071 | 0.042 |
| Example 22 | Compound 7 | Li | 99:1 | 0.069 | 0.035 |
| Example 23 | Compound 8 | Li | 99:1 | 0.064 | 0.033 |
| Example 24 | Compound 9 | Li | 99:1 | 0.075 | 0.041 |
| Example 25 | Compound 10 | Li | 99:1 | 0.084 | 0.052 |
| Example 26 | Compound 11 | Li | 99:1 | 0.081 | 0.030 |
| Example 27 | Compound 12 | Li | 99:1 | 0.086 | 0.059 |
| Comparative Example 1 | Compound 13 | Yb | 9:1 | 0.177 | 0.095 |
| Comparative Example 2 | Compound 14 | Yb | 9:1 | 0.157 | 0.101 |
| Comparative Example 3 | Compound 15 | Yb | 9:1 | 0.328 | 0.129 |
| Comparative Example 4 | Compound 16 | Yb | 9:1 | 0.294 | 0.152 |
| Comparative Example 5 | Compound 17 | Yb | 9:1 | 0.341 | 0.158 |
| Comparative Example 6 | Compound 18 | Yb | 9:1 | 0.417 | 0.178 |
| Comparative Example 7 | Compound 19 | Yb | 9:1 | 0.429 | 0.206 |
| Comparative Example 8 | Compound 13 | Yb | 99:1 | 0.172 | 0.092 |
| Comparative Example 9 | Compound 13 | Yb | 19:1 | 0.180 | 0.099 |
| Comparative Example 10 | Compound 13 | Li | 99:1 | 0.192 | 0.088 |
| Comparative Example 11 | Compound 14 | Li | 99:1 | 0.189 | 0.103 |
| Comparative Example 12 | Compound 15 | Li | 99:1 | 0.341 | 0.123 |
| Comparative Example 13 | Compound 16 | Li | 99:1 | 0.329 | 0.142 |
| Comparative Example 14 | Compound 17 | Li | 99:1 | 0.361 | 0.144 |
| Comparative Example 15 | Compound 18 | Li | 99:1 | 0.430 | 0.170 |
| Comparative Example 16 | Compound 19 | Li | 99:1 | 0.442 | 0.199 |

### Example 28

A glass substrate on which an ITO transparent electroconductive film was deposited as an anode in a thickness of 165 nm (manufactured by GEOMATEC Co., Ltd., 11 Ω/□, sputtered product) was cut into 38 mm × 46 mm, and etched. The resulting substrate was ultrasonically washed for 15 minutes using "Semico Clean" 56 (product name, manufactured by Furuuchi Chemical Corporation), and was then washed with ultra-pure water. This substrate was treated with UV-ozone for 1 hour immediately before preparation of an element, and placed in a vacuum deposition apparatus, and the apparatus was evacuated until the degree of vacuum in the apparatus was 5 × 10⁻⁴ Pa or less. By a resistance heating method, first, p-D1 was vapor-deposited as a hole injection layer in a thickness of 5 nm, and subsequently, HT-1 was vapor-deposited as a hole transporting layer in a thickness of 50 nm. Next, a mixed layer of host material H-1 and dopant material D-1 was vapor-deposited as an emissive layer in a thickness of 20 nm such that the doping concentration was 5% by weight. Next, ET-1 and 2E-1 were vapor-deposited as an electron transporting layer in a thickness of 35 nm such that the vapor deposition rate ratio was ET-1 : 2E-1 = 1 : 1. Compound 1 and a metal element Yb as a dopant were vapor-deposited as an electron injection layer in a thickness of 10 nm such that the vapor deposition rate ratio was Compound 1 : Yb = 9 : 1. Thereafter, aluminum was vapor-deposited in a thickness of 60 nm to form a cathode, and a 5 mm × 5 mm square organic EL element was produced.

This organic EL element was evaluated by the methods described above, and as a result, the initial driving voltage was 4.52 V, the external quantum efficiency (luminous efficiency) was 5.50%, the durable life was 960 hours, and the voltage build-up amount when being driven at room temperature for 100 hours was 0.021 V. p-D1, HT-1, H-1, D-1, ET-1, and 2E-1 are the compounds shown below.

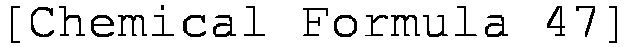

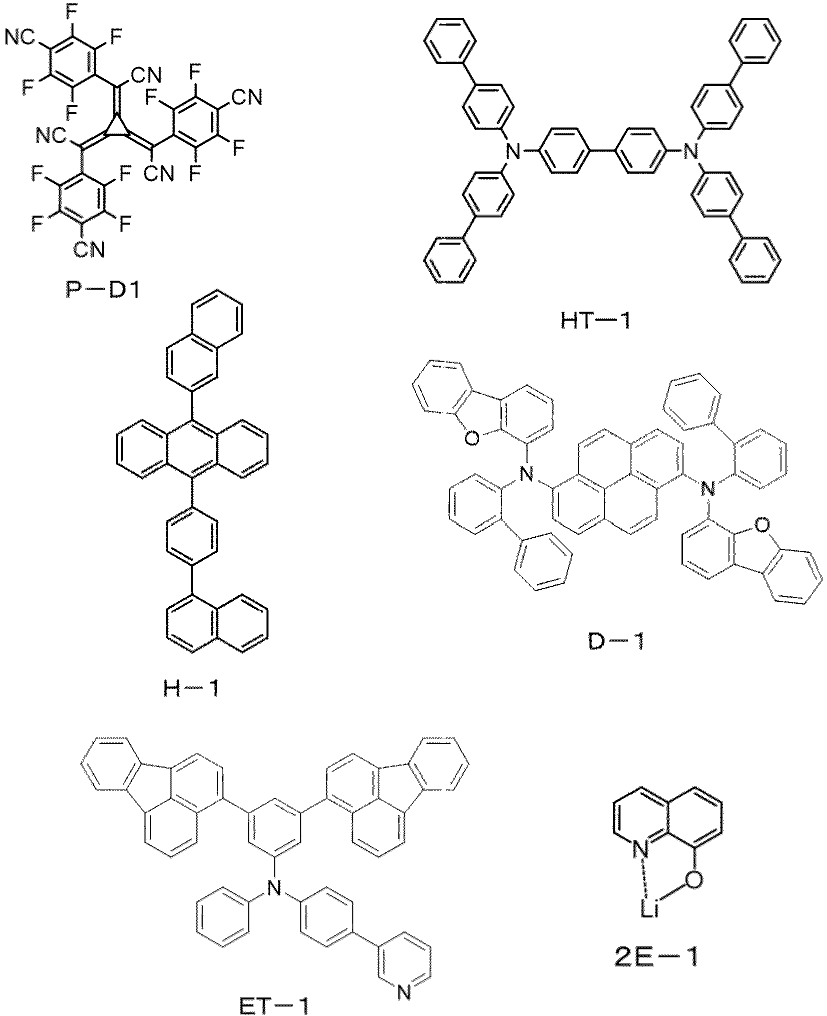

### Examples 29 to 54, Comparative Examples 17 to 32

Organic EL elements were produced in the same manner as in Example 28 except that the compound to be used, the metal element, and the vapor-deposition rate ratio of the compound to the metal element were varied as shown in Table 2. The results of the example and the comparative example are shown in Table 2.

**[Table 2]**

| | Compound | Metal element (Dopant) | Vapor deposition rate ratio, compound : metal element | Driving voltage (V) | External quantum efficiency (%) | Durability (h) | voltage build-up (V) at 10 mA/cm², 100 hours |
|---|---|---|---|---|---|---|---|
| Example 28 | Compound 1 | Yb | 9:1 | 4.52 | 5.50 | 960 | 0.021 |
| Example 29 | Compound 2 | Yb | 9:1 | 4.40 | 5.72 | 1040 | 0.024 |
| Example 30 | Compound 3 | Yb | 9:1 | 4.52 | 5.52 | 1000 | 0.016 |
| Example 31 | Compound 4 | Yb | 9:1 | 4.53 | 5.39 | 990 | 0.025 |
| Example 32 | Compound 5 | Yb | 9:1 | 4.38 | 5.70 | 1080 | 0.010 |
| Example 33 | Compound 6 | Yb | 9:1 | 4.41 | 5.75 | 1090 | 0.012 |
| Example 34 | Compound 7 | Yb | 9:1 | 4.37 | 5.69 | 1060 | 0.009 |
| Example 35 | Compound 8 | Yb | 9:1 | 4.18 | 5.78 | 1090 | 0.008 |
| Example 36 | Compound 9 | Yb | 9:1 | 4.45 | 5.81 | 1120 | 0.006 |
| Example 37 | Compound 10 | Yb | 9:1 | 4.47 | 5.69 | 950 | 0.021 |
| Example 38 | Compound 11 | Yb | 9:1 | 4.40 | 5.84 | 1150 | 0.006 |
| Example 39 | Compound 12 | Yb | 9:1 | 4.41 | 5.61 | 1010 | 0.022 |
| Example 40 | Compound 1 | Yb | 99:1 | 4.76 | 5.32 | 990 | 0.018 |
| Example 41 | Compound 1 | Yb | 19:1 | 4.62 | 5.41 | 970 | 0.024 |
| Example 42 | Compound 1 | Yb | 7:3 | 4.60 | 5.48 | 970 | 0.022 |
| Example 43 | Compound 1 | Li | 99:1 | 4.60 | 5.40 | 920 | 0.027 |
| Example 44 | Compound 2 | Li | 99:1 | 4.45 | 5.61 | 1000 | 0.028 |
| Example 45 | Compound 3 | Li | 99:1 | 4.61 | 5.41 | 990 | 0.022 |
| Example 46 | Compound 4 | Li | 99:1 | 4.59 | 5.30 | 960 | 0.031 |
| Example 47 | Compound 5 | Li | 99:1 | 4.48 | 5.57 | 1070 | 0.014 |
| Example 48 | Compound 6 | Li | 99:1 | 4.53 | 5.61 | 1050 | 0.019 |
| Example 49 | Compound 7 | Li | 99:1 | 4.49 | 5.62 | 1020 | 0.023 |
| Example 50 | Compound 8 | Li | 99:1 | 4.41 | 5.64 | 1090 | 0.014 |
| Example 51 | Compound 9 | Li | 99:1 | 4.57 | 5.50 | 1100 | 0.017 |
| Example 52 | Compound 10 | Li | 99:1 | 4.66 | 5.53 | 930 | 0.033 |
| Example 53 | Compound 11 | Li | 99:1 | 4.55 | 5.61 | 1100 | 0.012 |
| Example 54 | Compound 12 | Li | 99:1 | 4.60 | 5.41 | 960 | 0.031 |
| Comparative Example 17 | Compound 13 | Yb | 9:1 | 5.79 | 4.38 | 720 | 0.141 |
| Comparative Example 18 | Compound 14 | Yb | 9:1 | 6.12 | 4.26 | 700 | 0.153 |
| Comparative Example 19 | Compound 15 | Yb | 9:1 | 6.52 | 4.18 | 400 | 0.231 |
| Comparative Example 20 | Compound 16 | Yb | 9:1 | 6.61 | 4.09 | 370 | 0.197 |
| Comparative Example 21 | Compound 17 | Yb | 9:1 | 6.40 | 4.22 | 440 | 0.167 |
| Comparative Example 22 | Compound 18 | Yb | 9:1 | 7.32 | 3.58 | 480 | 0.210 |
| Comparative Example 23 | Compound 19 | Yb | 9:1 | 7.16 | 3.95 | 400 | 0.224 |
| Comparative Example 24 | Compound 13 | Yb | 99:1 | 6.01 | 4.53 | 750 | 0.152 |
| Comparative Example 25 | Compound 13 | Yb | 19:1 | 5.95 | 4.48 | 740 | 0.150 |
| Comparative Example 26 | Compound 13 | Li | 99:1 | 5.77 | 4.71 | 690 | 0.142 |
| Comparative Example 27 | Compound 14 | Li | 99:1 | 6.09 | 4.62 | 690 | 0.162 |
| Comparative Example 28 | Compound 15 | Li | 99:1 | 6.49 | 4.21 | 370 | 0.246 |
| Comparative Example 29 | Compound 16 | Li | 99:1 | 6.48 | 4.07 | 350 | 0.233 |
| Comparative Example 30 | Compound 17 | Li | 99:1 | 6.31 | 4.60 | 350 | 0.188 |
| Comparative Example 31 | Compound 18 | Li | 99:1 | 7.21 | 3.68 | 470 | 0.242 |
| Comparative Example 32 | Compound 19 | Li | 99:1 | 7.09 | 4.02 | 370 | 0.241 |

### Example 55

A glass substrate on which an ITO transparent electroconductive film was deposited as an anode in a thickness of 165 nm (manufactured by GEOMATEC Co., Ltd., 11 Ω/□, sputtered product) was cut into 38 mm × 46 mm, and etched. The resulting substrate was ultrasonically washed for 15 minutes using "Semico Clean" 56 (product name, manufactured by Furuuchi Chemical Corporation), and was then washed with ultra-pure water. This substrate was treated with UV-ozone for 1 hour immediately before preparation of an element, and placed in a vacuum deposition apparatus, and the apparatus was evacuated until the degree of vacuum in the apparatus was 5 × 10⁻⁴ Pa or less. First, p-D1 was vapor-deposited as a hole injection layer in a thickness of 5 nm by a resistance heating method. Subsequently, a light-emitting unit (first light-emitting unit) composed of a hole transporting layer, an emissive layer, and an electron transporting layer was formed on the hole injection layer.

Specifically, HT-1 was vapor-deposited in a thickness of 50 nm as the hole transporting layer, then a mixed layer of host material H-1 and dopant material D-1 was vapor-deposited in a thickness of 20 nm as the emissive layer such that the doping concentration was 5% by weight, and then ET-1 and 2E-1 were vapor-deposited in a thickness of 35 nm as the electron transporting layer such that the vapor deposition rate ratio was ET-1 : 2E-1 = 1 : 1.

Compound 1 and metal element Yb as a dopant were vapor-deposited as an N-type charge generation layer on the first light-emitting unit in a thickness of 10 nm at a vapor deposition rate ratio of Compound 1: Yb = 9 : 1, and then p-D1 was vapor-deposited as a p-type charge generation layer in a thickness of 10 nm.

Following the charge generation layer, a second light-emitting unit was formed in the same manner as the first light-emitting unit. Thereafter, Compound 1 and metal element Yb as a dopant were vapor-deposited as an electron injection layer in a thickness of 10 nm at a vapor-deposition rate ratio of Compound 1 : Yb = 9 : 1, and subsequently aluminum was vapor-deposited in a thickness of 60 nm to form a cathode, and thus a 5 mm × 5 mm square organic EL element was produced.

This organic EL element was evaluated by the methods described above, and as a result, the initial driving voltage was 9.14 V, the external quantum efficiency (luminous efficiency) was 11.03%, and the durable life was 1950 hours.

### Examples 56 to 81, Comparative Examples 33 to 48

Organic EL elements were produced in the same manner as in Example 55 except that the compound to be used and the vapor deposition rate ratio of the compound to the metal element were varied as shown in Table 3. The results of the example and the comparative example are shown in Table 3.

**[Table 3]**

| | N-type charge generation layer compound | Metal element (Dopant) | Vapor deposition rate ratio, compound : metal element | Driving voltage (V) | External quantum efficiency (%) | Durability (h) | Voltage build-up (V) at 10 mA/cm², 100 hours |
|---|---|---|---|---|---|---|---|
| Example 55 | Compound 1 | Yb | 9:1 | 9.14 | 11.03 | 1950 | 0.013 |
| Example 56 | Compound 2 | Yb | 9:1 | 8.92 | 11.32 | 2130 | 0.012 |
| Example 57 | Compound 3 | Yb | 9:1 | 9.23 | 11.10 | 2090 | 0.009 |
| Example 58 | Compound 4 | Yb | 9:1 | 9.10 | 10.49 | 2040 | 0.012 |
| Example 59 | Compound 5 | Yb | 9:1 | 8.89 | 11.28 | 2200 | 0.006 |
| Example 60 | Compound 6 | Yb | 9:1 | 8.93 | 11.57 | 2240 | 0.009 |
| Example 61 | Compound 7 | Yb | 9:1 | 8.74 | 11.29 | 2090 | 0.007 |
| Example 62 | Compound 8 | Yb | 9:1 | 8.42 | 11.31 | 2100 | 0.006 |
| Example 63 | Compound 9 | Yb | 9:1 | 9.16 | 11.15 | 2310 | 0.005 |
| Example 64 | Compound 10 | Yb | 9:1 | 9.05 | 11.20 | 1990 | 0.014 |
| Example 65 | Compound 11 | Yb | 9:1 | 8.92 | 11.45 | 2360 | 0.004 |
| Example 66 | Compound 12 | Yb | 9:1 | 8.89 | 10.93 | 2070 | 0.014 |
| Example 67 | Compound 1 | Yb | 99:1 | 9.56 | 10.64 | 1890 | 0.015 |
| Example 68 | Compound 1 | Yb | 19:1 | 9.31 | 10.88 | 1940 | 0.016 |
| Example 69 | Compound 1 | Yb | 7:3 | 9.22 | 10.74 | 1920 | 0.019 |
| Example 70 | Compound 1 | Li | 99:1 | 9.22 | 10.99 | 1980 | 0.021 |
| Example 71 | Compound 2 | Li | 99:1 | 9.03 | 11.21 | 2070 | 0.019 |
| Example 72 | Compound 3 | Li | 99:1 | 9.37 | 10.96 | 2030 | 0.015 |
| Example 73 | Compound 4 | Li | 99:1 | 9.21 | 10.40 | 1990 | 0.019 |
| Example 74 | Compound 5 | Li | 99:1 | 8.99 | 11.20 | 2020 | 0.009 |
| Example 75 | Compound 6 | Li | 99:1 | 9.06 | 11.42 | 2080 | 0.012 |
| Example 76 | Compound 7 | Li | 99:1 | 8.97 | 11.11 | 2060 | 0.017 |
| Example 77 | Compound 8 | Li | 99:1 | 8.92 | 11.10 | 2100 | 0.009 |
| Example 78 | Compound 9 | Li | 99:1 | 9.24 | 11.04 | 2170 | 0.014 |
| Example 79 | Compound 10 | Li | 99:1 | 9.18 | 10.88 | 1920 | 0.024 |
| Example 80 | Compound 11 | Li | 99:1 | 9.01 | 10.31 | 2250 | 0.009 |
| Example 81 | Compound 12 | Li | 99:1 | 9.44 | 10.59 | 1820 | 0.022 |
| Comparative Example 33 | Compound 13 | Yb | 9:1 | 11.52 | 8.56 | 1520 | 0.112 |
| Comparative Example 34 | Compound 14 | Yb | 9:1 | 12.40 | 8.32 | 1470 | 0.124 |
| Comparative Example 35 | Compound 15 | Yb | 9:1 | 13.52 | 8.10 | 820 | 0.193 |
| Comparative Example 36 | Compound 16 | Yb | 9:1 | 13.42 | 7.94 | 770 | 0.152 |
| Comparative Example 37 | Compound 17 | Yb | 9:1 | 12.90 | 8.33 | 910 | 0.130 |
| Comparative Example 38 | Compound 18 | Yb | 9:1 | 14.63 | 7.10 | 990 | 0.166 |
| Comparative Example 39 | Compound 19 | Yb | 9:1 | 14.93 | 7.56 | 810 | 0.172 |
| Comparative Example 40 | Compound 13 | Yb | 99:1 | 11.98 | 8.54 | 1400 | 0.089 |
| Comparative Example 41 | Compound 13 | Yb | 19:1 | 11.83 | 8.39 | 1420 | 0.133 |
| Comparative Example 42 | Compound 13 | Li | 99:1 | 11.67 | 9.21 | 1490 | 0.132 |
| Comparative Example 43 | Compound 14 | Li | 99:1 | 12.52 | 9.01 | 1300 | 0.142 |
| Comparative Example 44 | Compound 15 | Li | 99:1 | 13.49 | 8.22 | 770 | 0.232 |
| Comparative Example 45 | Compound 16 | Li | 99:1 | 13.55 | 8.01 | 740 | 0.172 |
| Comparative Example 46 | Compound 17 | Li | 99:1 | 12.84 | 9.01 | 900 | 0.142 |
| Comparative Example 47 | Compound 18 | Li | 99:1 | 14.70 | 7.35 | 920 | 0.189 |
| Comparative Example 48 | Compound 19 | Li | 99:1 | 14.88 | 7.14 | 760 | 0.123 |

In Examples 1 to 27, there were shown the results of single-charge elements in which a compound represented by the general formula (1) was used together with Li, which is an alkali metal element, or Yb, which is a rare earth metal element. On the other hand, in Comparative Examples 1 to 16, there were shown the results of light-emitting elements using Compounds 13 to 19, which are not compounds represented by the general formula (1), together with Li, which is an alkali metal element, or Yb, which is a rare earth metal element. In the Examples, the initial driving voltage was low and the increase in the driving voltage was small as compared with the Comparative Examples. This is understood as follows: Compounds 1 to 12 represented by the general formula (1) have stronger interaction such as coordination to an alkali metal element or a rare earth metal element as compared with Compounds 13 to 19, and have higher charge generation efficiency and electron transporting property, so that the carrier balance was stabilized, and the voltage build-up during driving could be controlled.

In addition, in Examples 1 to 15, there were shown results of single-charge elements containing a compound represented by the general formula (1) and Yb, which is a rare earth metal element, and in Examples 16 to 27, there were shown results of single-charge elements containing Li, which is an alkali metal element, instead of Yb. As understood from these results, it is found that a compound represented by the general formula (1) forms a stable layer with less increase in driving voltage as a result of use together with a rare earth metal element.

In Examples 28 to 54, there were shown the results of application of the organic layers used in Examples 1 to 27 to light-emitting elements. On the other hand, in Comparative Examples 17 to 32, there were shown the results of application of the organic layers used in Comparative Examples 1 to 16 to light-emitting elements. In the Examples, the driving voltage was low, the external quantum efficiency was high, the durability was improved, and the increase in the driving voltage was small as compared with the Comparative Examples. This indicates that similarly to the results of the single-charge elements, the compound represented by the general formula (1) is effective and forms a more stable light-emitting element exhibiting a small increase in the driving voltage.

In addition, in Examples 28 to 42, there were shown results of light-emitting elements containing a compound represented by the general formula (1) and Yb, which is a rare earth metal element, and in Examples 43 to 54, there were shown results of light-emitting elements containing Li, which is an alkali metal element, instead of Yb. As understood from these results, it is found that a compound represented by the general formula (1) forms a stable layer with less increase in driving voltage as a result of use together with a rare earth metal element.

In Examples 55 to 81, there were shown the results of application of the light-emitting elements used in Examples 28 to 54 to tandem type light-emitting elements. On the other hand, in Comparative Examples 33 to 48, there were shown the results of application of the light-emitting elements used in Comparative Examples 17 to 32 to tandem type light-emitting elements. In the Examples, the driving voltage was low, the external quantum efficiency was high, the durability was improved, and the increase in the driving voltage was small as compared with the Comparative Examples. This indicates that similarly to the results of the light-emitting elements used in Examples 28 to 54 and Comparative Examples 17 to 32, the compound represented by the general formula (1) is effective and forms a more stable tandem type light-emitting element exhibiting a small increase in the driving voltage.

In addition, in Examples 55 to 69, there were shown results of tandem type light-emitting elements containing a compound represented by the general formula (1) and Yb, which is a rare earth metal element, and in Examples 70 to 81, there were shown results of tandem type light-emitting elements containing Li, which is an alkali metal element, instead of Yb. As understood from these results, it is found that a compound represented by the general formula (1) forms a stable layer with less increase in driving voltage as a result of use together with a rare earth metal element.

## Claims

1. A compound represented by the following general formula (1), 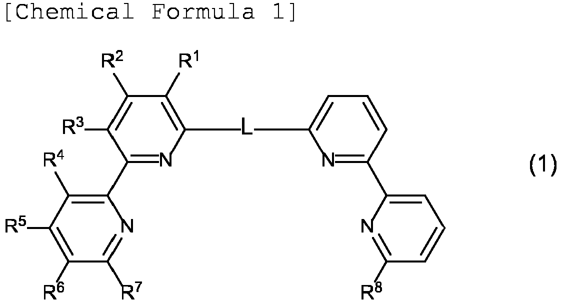 in the general formula (1), R¹, R², and R⁵ to R⁸ are each independently selected from the group consisting of a hydrogen atom, a substituted or unsubstituted alkyl group, a substituted or unsubstituted alkoxy group, a substituted or unsubstituted aryl group, and a substituted or unsubstituted heteroaryl group; R³ and R⁴ are each a carbon atom having a substituent; R³ and R⁴ may be bonded to each other; and L is an unsubstituted arylene group having 6 to 20 carbon atoms.

2. The compound according to claim 1, wherein in the general formula (1), R¹, R², R⁵ and R⁶ are each a hydrogen atom.

3. The compound according to claim 1, wherein in the general formula (1), R⁷ is a substituted or unsubstituted aryl group or a substituted or unsubstituted heteroaryl group.

4. The compound according to claim 1, wherein in the general formula (1), R³ and R⁴ are bonded to each other.

5. A light-emitting element in which at least an electron transporting layer and an emissive layer are present between an anode and a cathode and which emits light by electrical energy, wherein the electron transporting layer contains the compound according to any one of claims 1 to 4.

6. The light-emitting element according to claim 5, wherein the electron transporting layer further contains a complex composed of an alkali metal and an organic substance.

7. A light-emitting element in which at least a charge generation layer and an emissive layer are present between an anode and a cathode and which emits light by electrical energy, wherein the charge generation layer contains the compound according to any one of claims 1 to 4.

8. The light-emitting element according to claim 7, wherein the charge generation layer further contains a phenanthroline dimer.

9. The light-emitting element according to claim 7, wherein the charge generation layer further contains an alkali metal atom or a rare earth metal atom.

10. The light-emitting element according to claim 7, wherein the charge generation layer further contains an alkali metal atom, and the alkali metal atom is Li.

11. The light-emitting element according to claim 7, wherein the charge generation layer further contains a rare earth metal atom, and the rare earth metal atom is Yb.

12. A light-emitting element in which at least an electron injection layer and an emissive layer are present between an anode and a cathode and which emits light by electrical energy, wherein the electron injection layer contains the compound according to any one of claims 1 to 4.

13. The light-emitting element according to claim 12, wherein the electron injection layer further contains an alkali metal atom or a rare earth metal atom.

14. A display device comprising a light-emitting element containing the compound according to any one of claims 1 to 4.

15. An illumination device comprising a light-emitting element containing the compound of any one of claims 1 to 4.

## Patentansprüche

1. Verbindung, dargestellt durch die folgende allgemeine Formel (1), 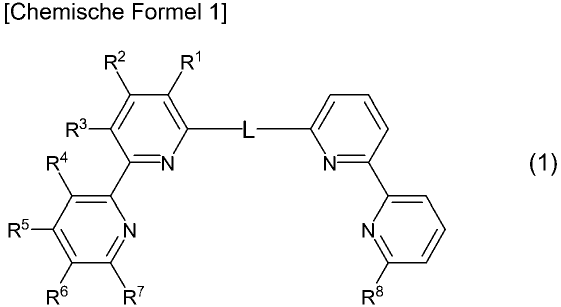 in der allgemeinen Formel (1) sind R¹, R² und R⁵ bis R⁸ jeweils unabhängig ausgewählt aus der Gruppe bestehend aus einem Wasserstoffatom, einer substituierten oder unsubstituierten Alkylgruppe, einer substituierten oder unsubstituierten Alkoxygruppe, einer substituierten oder unsubstituierten Arylgruppe und einer substituierten oder unsubstituierten Heteroarylgruppe; R³ und R⁴ sind jeweils ein Kohlenstoffatom mit einem Substituenten; R³ und R⁴ können aneinander gebunden sein; und L ist eine unsubstituierte Arylengruppe mit 6 bis 20 Kohlenstoffatomen.

2. Verbindung nach Anspruch 1, wobei in der allgemeinen Formel (1) R¹, R², R⁵ und R⁶ jeweils ein Wasserstoffatom sind.

3. Verbindung nach Anspruch 1, wobei in der allgemeinen Formel (1) R⁷ eine substituierte oder unsubstituierte Arylgruppe oder eine substituierte oder unsubstituierte Heteroarylgruppe ist.

4. Verbindung nach Anspruch 1, wobei in der allgemeinen Formel (1) R³ und R⁴ aneinander gebunden sind.

5. Licht emittierendes Element, in dem mindestens eine Elektronentransportschicht und eine Emissionsschicht zwischen einer Anode und einer Kathode vorhanden sind und das Licht durch elektrische Energie emittiert, wobei die Elektronentransportschicht die Verbindung nach einem der Ansprüche 1 bis 4 enthält.

6. Licht emittierendes Element nach Anspruch 5, wobei die Elektronentransportschicht ferner einen Komplex enthält, der aus einem Alkalimetall und einer organischen Substanz zusammengesetzt ist.

7. Licht emittierendes Element, in dem mindestens eine Ladungserzeugungsschicht und eine Emissionsschicht zwischen einer Anode und einer Kathode vorhanden sind und das Licht durch elektrische Energie emittiert, wobei die Ladungserzeugungsschicht die Verbindung nach einem der Ansprüche 1 bis 4 enthält.

8. Licht emittierendes Element nach Anspruch 7, wobei die Ladungserzeugungsschicht ferner ein Phenanthrolindimer enthält.

9. Licht emittierendes Element nach Anspruch 7, wobei die Ladungserzeugungsschicht ferner ein Alkalimetallatom oder ein Seltenerdmetallatom enthält.

10. Licht emittierendes Element nach Anspruch 7, wobei die Ladungserzeugungsschicht ferner ein Alkalimetallatom enthält und das Alkalimetallatom Li ist.

11. Licht emittierendes Element nach Anspruch 7, wobei die Ladungserzeugungsschicht ferner ein Seltenerdmetallatom enthält und das Seltenerdmetallatom Yb ist.

12. Licht emittierendes Element, in dem mindestens eine Elektroneninjektionsschicht und eine Emissionsschicht zwischen einer Anode und einer Kathode vorhanden sind und das Licht durch elektrische Energie emittiert, wobei die Elektroneninjektionsschicht die Verbindung nach einem der Ansprüche 1 bis 4 enthält.

13. Licht emittierendes Element nach Anspruch 12, wobei die Elektroneninjektionsschicht ferner ein Alkalimetallatom oder ein Seltenerdmetallatom enthält.

14. Anzeigevorrichtung, umfassend ein Licht emittierendes Element, das die Verbindung nach einem der Ansprüche 1 bis 4 enthält.

15. Beleuchtungsvorrichtung, umfassend ein Licht emittierendes Element, das die Verbindung nach einem der Ansprüche 1 bis 4 enthält.

## Revendications

1. Composé représenté par la formule générale (1) suivante, 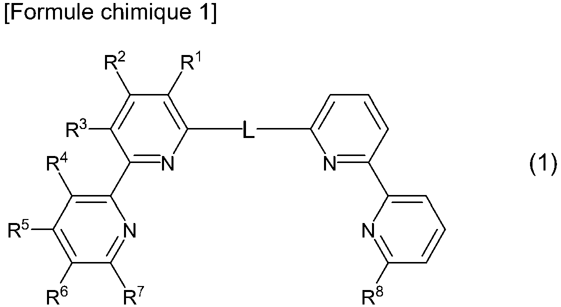 dans la formule générale (1), R¹, R², et R⁵ à R⁸ sont chacun indépendamment choisis dans le groupe constitué d'un atome d'hydrogène, d'un groupe alkyle substitué ou non substitué, d'un groupe alcoxy substitué ou non substitué, d'un groupe aryle substitué ou non substitué, et d'un groupe hétéroaryle substitué ou non substitué ; R³ et R⁴ sont chacun un atome de carbone ayant un substituant ; R³ et R⁴ peuvent être liés l'un à l'autre ; et L est un groupe arylène non substitué ayant 6 à 20 atomes de carbone.

2. Composé selon la revendication 1, dans lequel dans la formule générale (1), R¹, R², R⁵ et R⁶ sont chacun un atome d'hydrogène.

3. Composé selon la revendication 1, dans lequel dans la formule générale (1), R⁷ est un groupe aryle substitué ou non substitué ou un groupe hétéroaryle substitué ou non substitué.

4. Composé selon la revendication 1, dans lequel dans la formule générale (1), R³ et R⁴ sont liés l'un à l'autre.

5. Élément électroluminescent dans lequel au moins une couche de transport d'électrons et une couche émissive sont présentes entre une anode et une cathode et qui émet de la lumière par énergie électrique, dans lequel la couche de transport d'électrons contient le composé selon l'une quelconque des revendications 1 à 4.

6. Élément électroluminescent selon la revendication 5, dans lequel la couche de transport d'électrons contient en outre un complexe composé d'un métal alcalin et d'une substance organique.

7. Élément électroluminescent dans lequel au moins une couche de génération de charge et une couche émissive sont présentes entre une anode et une cathode et qui émet de la lumière par énergie électrique, dans lequel la couche de génération de charge contient le composé selon l'une quelconque des revendications 1 à 4.

8. Élément électroluminescent selon la revendication 7, dans lequel la couche de génération de charge contient en outre un dimère de phénanthroline.

9. Élément électroluminescent selon la revendication 7, dans lequel la couche de génération de charge contient en outre un atome de métal alcalin ou un atome de métal de terres rares.

10. Élément électroluminescent selon la revendication 7, dans lequel la couche de génération de charge contient en outre un atome de métal alcalin, et l'atome de métal alcalin est Li.

11. Élément électroluminescent selon la revendication 7, dans lequel la couche de génération de charge contient en outre un atome de métal de terres rares, et l'atome de métal de terres rares est Yb.

12. Élément électroluminescent dans lequel au moins une couche d'injection d'électrons et une couche émissive sont présentes entre une anode et une cathode et qui émet de la lumière par énergie électrique, dans lequel la couche d'injection d'électrons contient le composé selon l'une quelconque des revendications 1 à 4.

13. Élément électroluminescent selon la revendication 12, dans lequel la couche d'injection d'électrons contient en outre un atome de métal alcalin ou un atome de métal de terres rares.

14. Dispositif d'affichage comprenant un élément électroluminescent contenant le composé selon l'une quelconque des revendications 1 à 4.

15. Dispositif d'éclairage comprenant un élément électroluminescent contenant le composé selon l'une quelconque des revendications 1 à 4.
